# EUROPEAN PATENT SPECIFICATION

(11) **EP 2 978 863 B1**
(45) Date of publication and mention of the grant of the patent: **10.03.2021**
(21) Application number: 14773528.6
(22) Date of filing: 28.03.2014
(51) Int. Cl.: C12Q 1/70, G01N 33/53

(54) **DIFFERENTIATION BETWEEN TRANSIENT AND PERSISTENT HIGH RISK HPV INFECTION BY IN SITU HYBRIDIZATION**
DIFFERENZIERUNG ZWISCHEN TRANSIENTEM UND PERSISTENTEM HOHEM RISIKO EINER HPV-INFEKTION DURCH IN SITU-HYBRIDISIERUNG
DIFFÉRENCIATION ENTRE UNE INFECTION PAR LE HPV À HAUT RISQUE TRANSITOIRE ET PERSISTANTE PAR HYBRIDATION IN SITU

(30) Priority: 28.03.2013 US 201361806360 P
(43) Date of publication of application: 03.02.2016
(73) Proprietor: Advanced Cell Diagnostics, Inc., Newark, CA 94560 (US)
(72) Inventor: MA, Xiao-Jun, Hayward, CA 94545 (US); WANG, Hongwei, Hayward, CA 94545 (US); WU, Xingyong, Hayward, CA 94545 (US); LUO, Yuling, Hayward, CA 94545 (US)
(74) Representative: Glawe, Delfs, Moll
(86) International application number: PCT/US2014/032195
(87) International publication number: WO 2014/160949

(56) References cited:
- WO-A1-2005/083129
- WO-A1-2010/129941
- WO-A2-2012/103414
- US-A1- 2003 190 602
- US-A1- 2010 120 019
- US-A1- 2012 214 152
- US-A1- 2012 225 792
- DE MARCHI TRIGLIA R ET AL: "HPV in situ hybridization signal patterns as a marker for cervical intraepithelial neoplasia progression", GYNECOLOGIC ONCOLOGY, ACADEMIC PRESS, LONDON, GB, vol. 112, no. 1, 1 January 2009 (2009-01-01), pages 114-118, XP025845616, ISSN: 0090-8258, DOI: 10.1016/J.YGYNO.2008.09.047 [retrieved on 2008-11-12]
- M. GUO ET AL: "Evaluation of a Commercialized In Situ Hybridization Assay for Detecting Human Papillomavirus DNA in Tissue Specimens from Patients with Cervical Intraepithelial Neoplasia and Cervical Carcinoma", JOURNAL OF CLINICAL MICROBIOLOGY, vol. 46, no. 1, 1 January 2008 (2008-01-01), pages 274-280, XP55311937, US ISSN: 0095-1137, DOI: 10.1128/JCM.01299-07
- ODEY C. UKPO ET AL: "High-Risk Human Papillomavirus E6/E7 mRNA Detection by a Novel In Situ Hybridization Assay Strongly Correlates With p16 Expression and Patient Outcomes in Oropharyngeal Squamous Cell Carcinoma", AMERICAN JOURNAL OF SURGICAL PATHOLOGY., vol. 35, no. 9, 1 September 2011 (2011-09-01), pages 1343-1350, XP055310867, US ISSN: 0147-5185, DOI: 10.1097/PAS.0b013e318220e59d
- ANTON H.N. HOPMAN ET AL: "HPV in situ hybridization: Impact of different protocols on the detection of integrated HPV", INTERNATIONAL JOURNAL OF CANCER, vol. 115, no. 3, 1 February 2005 (2005-02-01), pages 419-428, XP055145625, ISSN: 0020-7136, DOI: 10.1002/ijc.20862
- Dr Alinda ET AL: "Combination of cytological and molecular methods for improvements in cervical cancer prevention PhD Dissertation", , 1 January 2007 (2007-01-01), XP055311543, Retrieved from the Internet: URL:http://aok.pte.hu/docs/phd/file/dolgoz atok/2007/Varnai_Alinda_Dalma_PhD_dolgozat .pdf [retrieved on 2016-10-18]
- Mark F. Evans ET AL: "HPV E6/E7 RNA In Situ Hybridization Signal Patterns as Biomarkers of Three-Tier Cervical Intraepithelial Neoplasia Grade", PLoS ONE, vol. 9, no. 3, 13 March 2014 (2014-03-13), page e91142, XP055311296, DOI: 10.1371/journal.pone.0091142

## Description

This application claims the benefit of priority of United States Provisional application serial No. 61/806,360, filed March 28, 2013.

### BACKGROUND OF THE INVENTION

The present invention relates generally to *in situ* hybridization assays and cervical sample analysis, and more specifically to assays for testing for human papillomavirus (HPV) infection and analysis of cervical tissue samples.

High-risk human papillomavirus (HR-HPV) infection causes essentially all cervical cancer and a subset of head and neck cancer (Zur Hausen, Virology 384:260-265 (2009)). However, HPV infection alone is not sufficient for oncogenic transformation; 80-90% of infections are transient and self-cleared by the immune system. Current HPV testing methods such as Hybrid Capture® 2 (Digene Corporation; Gaithersburg, MD) and GP5+/6+ PCR have excellent analytical sensitivity but do not differentiate transient from persistent HPV infections, only reporting HPV positivity (Kroupis and Vourlidis, Clin. Chem. Lab. Med. 49:1783-1799 (2011)). As a result, these tests have poor clinical specificity (40-50%) for high-grade intraepithelial neoplasms (CIN2+), leading to unnecessary follow-up procedures for many women (Kinney et al., Am. J. Clin. Pathol. 134:193-199 (2010)). Therefore, there is an unmet need for an HPV assay that is both analytically sensitive and clinically specific.

Thus, there exists a need for methods to accurately assess and categorize cervical tissue samples based on the presence of HPV nucleic acids. The present invention satisfies this need and provides related advantages as well.

### SUMMARY OF INVENTION

The scope of the invention is defined by the appended claims. The invention relates to methods of categorizing a cervical tissue or cytology sample by performing an *in situ* hybridization assay using an antisense E6 or E7 probe on a cervical tissue sample, wherein the antisense E6 or E7 probe can simultaneously detect HPV DNA and HPV RNA; detecting the presence of HPV nucleic acid; and categorizing the cervical tissue sample based on HPV nucleic acid expression.

### BRIEF DESCRIPTION OF THE DRAWINGS

Figure 1 shows serial sections of a formalin-fixed paraffin-embedded block of cultured Caski cells. Caski cells were stained for UBC, encoding ubiquitin (endogenous RNA control) (left column), antisense probe against HPV16 E6/E7 mRNA (middle column), and the sense probe against HPV16 E6/E7 DNA (right column). Top row, standard conditions (no DNase). Bottom row, sections were pretreated with DNase then hybridized with HPV probes. RNAscope® staining signals are in brown color (DAB). Nuclei are stained with hematoxylin (blue).

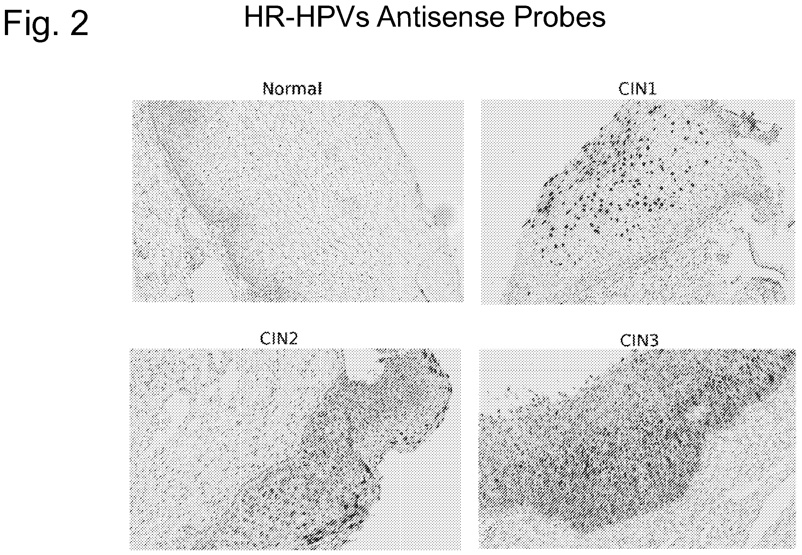

Figure 2 shows HPV E6/E7 staining patterns in normal cervix and HPV-associated cervical lesions of different histological grade. Cervical biopsies were stained for high risk HPV E6/E7 using a pool of probes against 18 subtypes (16, 18, 26, 31, 33, 35, 39, 45, 51, 52, 53, 56, 58, 59, 66, 68, 73 and 82). Normal cervix was completely negative for high-risk HPV. Note that CIN1 had strong staining in the upper 1/3 layer of the cervical epithelium, but its staining pattern is different from that of CIN3, where the signals appeared as fine brown granules throughout the epithelium. Some CIN2 exhibit an intermediate pattern as shown. RNAscope® staining signals are in brown color (DAB). Nuclei are stained with hematoxylin (blue).
Figure 3 shows a CIN2 lesion stained with the antisense probes targeting high-risk HPVs or the corresponding sense probes. RNAscope® staining signals are in brown color (DAB). Nuclei are stained with hematoxylin (blue).
Figure 4 shows a CIN2 lesion stained with the antisense probes targeting high-risk HPVs or the corresponding sense probes. Upper, 20x lens; lower 40X lens. RNAscope® signals are in brown color (DAB). Nuclei are stained with hematoxylin (blue).
Figure 5 shows the same CIN2 lesion as in Figure 3 pretreated with an RNase digest then stained with the antisense probes targeting high-risk HPVs or the corresponding sense probes. Upper, 20x lens; lower 40X lens. RNAscope® signals are in brown color (DAB). Nuclei are stained with hematoxylin (blue).
Figure 6 shows the same CIN2 lesion as in Figure 3 pretreated with DNase then stained with the sense HPV probes. Left, 20x lens; right, 40X lens. RNAscope® signals are in brown color (DAB). Nuclei are stained with hematoxylin (blue).
Figure 7 shows a schematic diagram of the layers of the cervical epithelium.

### DETAILED DESCRIPTION OF THE INVENTION

The invention relates to assays and methods for classifying the status of cervical tissue samples with respect to human papillomavirus (HPV). High-risk human papillomavirus (HR-HPV) infection causes essentially all cervical cancer. The methods disclosed herein allow cervical tissue samples to be classified or categorized as to the likelihood of the sample containing a low-grade lesion, which is most likely to be self-clearing, or a high-grade lesion (considered precancerous), which has an increased likelihood of progressing to a cancerous state. The methods of the invention can be used alone or in combination with known histological screening methods, such as from a Pap smear, to classify or categorize a cervical tissue sample. The methods of the invention permit a determination as to whether no treatment is indicated or whether a treatment regimen should be implemented with an individual. The methods of the invention are based on differentiating between transient high-risk HPV infection, which is more likely to spontaneously clear, and a persistent high-risk HPV infection, which has an increased likelihood of progressing to a cancerous state.

As used herein, the term "nucleic acid," or alternatively the term "polynucleotide," refers to a polymer of nucleotide monomer units, as is well known to those skilled in the art. Exemplary nucleic acids include, for example, DNA and RNA, including mRNA, siRNA, hnRNA, genomic DNA, cDNA, or other well known forms of nucleic acids. The nucleic acid or polynucleotide can contain naturally occurring nucleotides, including well known naturally occurring nucleotide modifications, as well as non-naturally occurring nucleotides such as those made by chemical synthesis. Such modifications include, but are not limited to, peptide nucleic acids (PNAs), modified oligonucleotides, for example, oligonucleotides comprising nucleotides that are not typical to biological RNA or DNA, such as 2'-O-methylated oligonucleotides, and the like. The nucleotides of the polynucleotide can be deoxyribonucleotides, ribonucleotides or nucleotide analogs, can be natural or non-natural, and can be unsubstituted, unmodified, substituted or modified. The nucleotides can be linked by phosphodiester bonds, or by phosphorothioate linkages, methylphosphonate linkages, boranophosphate linkages, or the like. The polynucleotide can additionally comprise non-nucleotide elements such as labels, quenchers, blocking groups, or the like, as disclosed herein. The polynucleotide can be single-stranded or double-stranded.

As disclosed herein, the term "HPV nucleic acid" when used in reference to a sample refers to HPV DNA or RNA, in particular mRNA, or a combination of HPV DNA and HPV RNA such as HPV mRNA.

As used herein, a "nucleic acid target" or "target nucleic acid" refers to a nucleic acid, or a region thereof, that is intended to be detected. One skilled in the art can readily determine target nucleic acids desired to be detected by methods of the invention. As described herein, the invention relates to methods of detecting HPV nucleic acids, in particular high risk HPVs, as disclosed herein. Therefore, HPV nucleic acids, in particular high-risk HPV nucleic acids, are target nucleic acids of particular interest in the methods of the invention, as disclosed herein.

As used herein, a "label" is a moiety that facilitates detection of a molecule. Common labels in the context of the present invention include fluorescent, luminescent, light-scattering, and/or colorimetric labels. Suitable labels include enzymes, and fluorescent and chromogenic moieties, as well as radionuclides, substrates, cofactors, inhibitors, chemiluminescent moieties, magnetic particles, and the like. In a particular embodiment of the invention, the label is an enzyme. Exemplary enzyme labels include, but are not limited to Horse Radish Peroxidase (HRP), Alkaline Phosphatase (AP), β-galactosidase, glucose oxidase, and the like, as well as various proteases. Other labels include, but are not limited to, fluorophores, Dinitrophenyl (DNP), and the like. Labels are well known to those skilled in the art, as described, for example, in Hermanson, Bioconjugate Techniques, Academic Press, San Diego (1996), and U.S. Patent Nos. 3,817,837; 3,850,752; 3,939,350; 3,996,345; 4,277,437; 4,275,149; and 4,366,241. Many labels are commercially available and can be used in methods and assays of the invention, including detectable enzyme/substrate combinations (Pierce, Rockford IL; Santa Cruz Biotechnology, Dallas TX; Invitrogen, Carlsbad CA). In a particular embodiment of the invention, the enzyme can utilize a chromogenic or fluorogenic substrate to produce a detectable signal, as described herein. Exemplary labels are described herein.

As used herein, *"in situ* hybridization" or "ISH" refers to a type of hybridization that uses a directly or indirectly labeled complementary DNA or RNA strand, such as a probe, to bind to and localize a specific nucleic acid, such as DNA or RNA, in a sample, in particular a portion or section of tissue (*in situ*)*.* The probe types can be double stranded DNA (dsDNA), single stranded DNA (ssDNA), single stranded complimentary RNA (sscRNA), messenger RNA (mRNA), micro RNA (miRNA), and/or synthetic oligonucleotides. The term "fluorescent *in situ* hybridization" or "FISH" refers to a type of ISH utilizing a fluorescent label. The term "chromogenic *in situ* hybridization" or "CISH" refers to a type of ISH with a chromogenic label. ISH, FISH and CISH methods are well known to those skilled in the art (see, for example, Stoler, Clinics in Laboratory Medicine 10(1):215-236 (1990); In situ hybridization. A practical approach, Wilkinson, ed., IRL Press, Oxford (1992); Schwarzacher and Heslop-Harrison, Practical in situ hybridization, BIOS Scientific Publishers Ltd, Oxford (2000)).

As disclosed herein, the ability to simultaneously detect both viral DNA and RNA signals from HPV infected cells using the antisense E6/E7 probes, optionally assisted by the use of corresponding sense probes in a parallel assay, allows differentiation between transient and persistent HPV infections. This distinction can have important clinical applications since it can allow cervical lesions to be classified by the state of HPV infection into three stages - transient state (CIN1-like pattern), transition state (hybrid pattern), and persistent state (CIN3-like pattern) - as opposed to morphological criteria alone.

The methods of the invention can be used to assess disease state and risk of progression using cervical biopsies. The CIN1 and CIN3 signal patterns and the hybrid pattern represent three different stages of disease progression in relation to HPV infection. The CIN1 pattern appears to indicate a transient HPV infection, whereas a CIN3 pattern appears to indicate the establishment of persistent infection. The hybrid pattern appears to indicate a transition state when the virus starts to establish persistence in the basal layer. Therefore, both the hybrid and CIN3 patterns appear to indicate a high risk lesion, whereas the CIN1 pattern indicates a low risk lesion, which is likely self-clearing.

Classification of cervical lesions by the HPV DNA/RNA staining pattern has advantages over traditional histological CIN grading. For example, the existence of CIN2 has been considered as a diagnostic ambiguity as opposed to a true disease state (Galgano et al., Am. J. Surg. Pathol. 34:1077-1087 (2010)), thus leading to a treatment dilemma or overtreatment. In contrast, the hybrid pattern observed in the experiments disclosed herein can be more informative for treatment decision making, since it may indicate the beginning of viral persistence and thus should be treated like a persistent state. The HPV DNA/RNA staining pattern can also allow reclassification of some CIN1 lesions into high risk lesions, allowing earlier treatment and improved outcome while sparing patients with truly low risk CIN1 lesions aggressive follow up and/or treatment procedures. Likewise, histologically CIN2 and CIN3 lesions may also be reclassified by the state of HPV infection and treated according to the state of viral persistence. In addition, different levels of HPV E6/E7 RNA or the relative amounts of HPV E6/E7 mRNA and DNA can be associated with different levels of risk of developing high grade CIN lesions and invasive cervical cancers (prognosis), again allowing more personalized management.

The methods of the invention can also be used to assess disease state and risk of progression using cytology samples. Similar to the situation in cervical biopsies, a strong diffusedly stained nucleus in a cell by either the antisense or the sense probe can indicate a transiently infected cell, whereas the presence of cytoplasmic staining using the antisense probes can indicate a persistently infected cell, for example, through viral integration into the host genome. Again, recognition of these two types of HPV-infected cells and their absolute or relative cell numbers and/or the relative levels of HPV E6/E7 mRNA and DNA can be used to indicate the presence of low or high grade lesions or to predict risk of disease progression (prognosis).

It is known that certain HPV subtypes are correlated with an increased likelihood of developing cervical cancer, whereas other HPV subtypes are associated with a low risk of developing cervical cancer (see, for example, Burd, Clin. Microbio. Rev. 16:1-17 (2003)). In a patient who is diagnosed with having cervical lesions, determining the presence of E6/E7 mRNA of one or more high risk HPV (HR-HPV) can be used determine whether the cervical lesions are benign or related to CIN (see WO 2012/103414; US publication 20120214152). As used herein, the following HPV subgroup of 18 subtypes are considered to be high-risk HPV subtypes. These high-risk HPV subtypes include, but are not limited to, HPV-16, 18, 26, 31, 33, 35, 39, 45, 51, 52, 53, 56, 58, 59, 66, 68, 73, and 82, and this group is also referred to herein as the HPV subgroup of 18. If desired, a group of fifteen HPV subtypes including HPV-16, 18, 31, 33, 35, 39, 45, 51, 52, 56, 58, 59, 68 and 73, also referred to herein as the subgroup of 15, can also be used in the test of a cervical lesion. An individual HPV subtype in the HPV subgroup of 18, or a combination of one or more of the 18 subtypes, are considered to be high-risk HPV subtypes to be analyzed in the methods disclosed herein. Another group of high-risk HPV subtypes can be a group including HPV-16 and 18. Still another group of high-risk HPV subtypes can be a group including HPV-16, 18, 31, 33, 45, 52 and 58. It is understood that any one or more of the high-risk HPV subtypes selected from HPV-16, 18, 26, 31, 33, 35, 39, 45, 51, 52, 53, 56, 58, 59, 66, 68, 73, and 82 can be used as nucleic acid targets to detect high-risk HPV, for example, as a probe directed to the E6 and/or E7 region of any one or more of the HPV high-risk HPV subtypes in the methods of the invention. Thus, in an embodiment of the invention, a probe comprises target probe sets complementary to the E6 and/or E7 region of one or more of the HPV subtypes selected from 16, 18, 26, 31, 33, 35, 39, 45, 51, 52, 53, 56, 58, 59, 66, 68, 73, and 82, including up to all of the HPV subtypes. In another embodiment, the probe comprises probe sets complementary to the E6 and/or E7 region of one or more of the HPV subtypes 16, 18, 31, 33, 35, 39, 45, 51, 52, 56, 58, 59, 68 and 73, including up to all of the HPV subtypes. In still another embodiment, the probe comprises probe sets complementary to the E6 and/or E7 region of one or more of the HPV subtypes 16, 18, 31, 33, 45, 52 and 58, including up to all of the HPV subtypes. In another embodiment the probe comprises probe sets complementary to the E6 and/or E7 region of the HPV subtypes 16 and/or 18.

The sequences of numerous HPV subtypes are known in the art and can be readily identified by those skilled in the art. Exemplary sources for sequences of HPV subtypes can be found, for example, at the National Center for Biotechnology Information (NCBI)(ncbi.nlm.nih.gov). Exemplary HPV subtype sequences can be found, for example, at the NCBI GenBank database and include, but are not limited to, representative genomic sequences for HPV-16 (NC_001526.2; GI:310698439); HPV-18 (AY262282.1; GI:30172004), HPV-26 (NC_001583.1; GI:9627305), HPV-31 (J04353.1; GI:333048), HPV-33 (M12732.1; GI:333049), HPV-35 (M74117.1; GI:333050), HPV-39 (KC470248.1; GI:537802154), HPV-45 (KC470260.1; GI:537802294), HPV-51 (M62877.1; GI:333087; Lungu et al., J. Virol. 65:4216-4225 (1991)), HPV-52 (37751.1; GI:337238732), HPV-53 (NC_001593.1; GI:9627377), HPV-56 (EF177181.1; GI:138374823), HPV-58 (D90400.1; GI:222386), HPV-59 (X77858.1; GI:557236), HPV-66 (U31794.1; GI:1020290), HPV-68 (KC470283.1; GI:537802536), HPV-73 (X94165.1; GI:1491692), and HPV-82 (AB027021.1; GI:6970427). Other representative sequences of the various high-risk HPV subtypes are also available at the NCBI GenBank database.

Such genomic sequences available at the NCIB GenBank database include, for example, annotation indicating the position of E6 and E7 genes. Therefore, one skilled in the art can readily determine and identify suitable sequences to use as sense or antisense probes for the E6 and E7 encoding regions using routine methods well known to those skilled in the art (see also Burd et al., *supra,* 2003, for a description of HPV genome organization and encoded genes). Such a selection of appropriate regions and design of specific and selective reagents that bind to the target nucleic acids, in particular oligonucleotides or probes that specifically and selectively bind to a target nucleic acid, are well known to those skilled in the art (see Sambrook et al., Molecular Cloning: A Laboratory Manual, Third Ed., Cold Spring Harbor Laboratory, New York (2001); Ausubel et al., Current Protocols in Molecular Biology, John Wiley and Sons, Baltimore, MD (1999). A desired specificity can be achieved using appropriate selection of regions of a target nucleic acid as well as appropriate lengths of a binding agent such as an oligonucleotide or probe, and such selection methods are well known to those skilled in the art. Thus, one skilled in the art will readily understand and can readily determine appropriate reagents, such as oligonucleotides or probes, that can be used to target one particular target nucleic acid over another target nucleic acid.

In one embodiment of the invention, an *in situ* hybridizaton (ISH) assay is used to detect the presence of E6 and/or E7 mRNA and/or DNA of one or more high-risk HPV subtypes in a tissue section or cytological sample derived from the cervix of a female individual. The tissue sample is obtained from an individual, for example, using an examination procedure such as a colposcopy or can be from a cervical tissue biopsy. Cytological samples can be derived from the cervix using a routine examination procedure such as a Pap smear or similar technique. Optionally, the sample can have been previously categorized using well known histological methods and/or previously identified as being HPV positive. The sample can be captured in a formalin fixed, paraffin embedded (FFPE) section, applied on a solid surface such as a slide, or suspended in a solution, such as in a cytological smear, and optionally applied to a solid support such as a slide. When an FFPE tissue section is used, the tissue can optionally be treated with a tissue pretreatment agent, such as being heated in a citrate buffer followed by digestion with a protease, or using other well known methods for processing FFPE for an ISH assay. In a particular embodiment, the tissue or cytology sample is pretreated, for example, with heat and/or buffers, to facilitate hybridization to DNA. After the sample is prepared, an ISH assay is performed to detect the high-risk HPV subtypes in the sample.

In the ISH HPV assay used in methods of the invention, generally a plurality of target probe sets are designed to hybridize to a plurality of target nucleic acids within the region of E6 and/or E7 mRNA or DNA of high-risk HPV subtypes. It is understood that using the term "target probe" for assessing the presence of HPV nucleic acid in a cervical sample using methods of the invention can refer to a single probe or can include a plurality of target probe sets to one or more high-risk HPV subtypes. In a particular embodiment, the plurality of target nucleic acids can include the HPV subgroup of 18. In another embodiment, the plurality of target nucleic acids can include the HPV subgroup of 15. In still another embodiment, the target nucleic acid can be HPV-16 alone. Each target probe set is designed to recognize one HPV subtype, and the target probe sets can be used individually or in a pool, depending on whether the HPV subtypes are intended to be detected individually or as a group together (indistinguishably). In a particular embodiment, each target probe set can contain an identical recognition sequence suitable for being detected by a universal signal amplification system that detects all target probes in the set, as described herein. Such a universal amplification system as used herein is generally an amplification system where the same label is used to detect all high risk HPV subtypes in the target probe set.

Traditionally, cervical tissue specimens, for example, as in a Pap smear or cervical tissue biopsy, are graded based on cytology or histology, respectively. The cervical tissue specimen is observed by a clinician, who grades the specimen by their morphology. Such histological grading method of cervical tissue specimens are well known in the art (see, for example, Cecil Textbook of Medicine, Bennett and Plum, eds., 20th ed., WB Saunders, Philadelphia (1996); Colposcopy and Treatment of Cervical Intraepithelial Neoplasia: A Beginner's Manual, Sellors and Sankaranarayanan, eds., International Agency for Research on Cancer, Lyon, France (2003); Kalaf and Cooper, J. Clin. Pathol. 60:449-455 (2007); Brown and Trimble, Best Pract. Res. Clin. Obstet. Gynaecol. 26:233-242 (2012); Waxman et al., Obstet. Gynecol. 120:1465-1471 (2012)). A table showing various terminology used in the grading of cervical samples is shown in Table 1.

**Table 1. Cervical Cell Grading Categories (Sellors and Sankaranarayanan, supra, 2003)**

| Dysplasia terminology | Original CIN terminology | Modified CIN terminology | The Bethesda system (SIL) terminology |
|---|---|---|---|
| Normal | Normal | Normal | Within normal limits Benign cellular changes (infection or repair) ASCUS/AGUS |
| Atypia | Koilocytic atypia, flat condyloma, without epithelial changes | Low-grade CIN | LSIL |
| Mild dysplasia or mild dyskaryosis | CIN 1 | Low-grade CIN | LSIL |
| Moderate dysplasia or moderate dyskaryusis | CIN 2 | High-grade CIN | HSIL |
| Severe dysplasia or severe dyskaryosis | CIN 3 | High-grade CIN | HSIL |
| Carcinoma *in-situ* | CIN 3 | High-grade CIN | HSIL |
| Invasive carcinoma | Invasive carcinoma | Invasive carcinoma | Invasive carcinoma |

| | | | |
|---|---|---|---|
| CIN: cervical intraepithelial neoplasia; LSIL: Low-grade squamous intraepithelial lesion; HSIL: High-grade squamous intraepithelial lesion; ASCUS: Atypical squamous cells of undetermined significance; AGUS: Atypical glandular cells of undetermined significance | | | |

Historically, both a three grade system, CIN1, CIN2 and CIN3, and a two grade system, LSIL and HSIL, have been used to categorize cervical tissue samples based on histology of cells or tissues other than normal tissue (see Table 1). When CIN grade or other histological criteria are used to grade a cervical tissue sample, the reliability of the diagnosis can be problematic since the histological diagnosis of CIN relies on subjective interpretation of cellular findings and morphology. Poor diagnostic accuracy and reproducibility can complicate and affect patient care. Incorrect classification of CIN grade on biopsy can lead to inappropriate follow-up or treatment of patients. Clinicians risk over-treating benign lesions or mismanaging precancerous lesions. The present invention provides methods to assist in the evaluation and diagnosis of cervical tissue specimens based on the presence of HPV high-risk subtype nucleic acids and determination of HPV infection state.

Currently, Pap (Papanicolaou) smears or liquid-based cytology material (for example, ThinPrep; Hologic; Bedford MA) obtained during cervical cancer screening suffer from the same limitations as processing of cervical biopsy on histology. Morphology of cervical cells based on cytology generally cannot be used to conclude whether the cause may relate to HPV infection. In addition, morphological changes due to benign HPV infections cannot be distinguished from malignant, precancerous HPV lesions. An advantage of the method disclosed herein is that it can test the nature of the cervical lesion on a molecular level, and thus determine whether the cervical lesion is likely to be related to a transient, persistent or transitional HPV infection, as disclosed herein.

The methods of the invention disclosed herein can used to determine a spatial pattern of expression of E6 and/or E7 mRNA and/or DNA and optionally levels of E6 and/or E7 mRNA and/or DNA of one or more high-risk HPV subtypes in cervical tissue sample, including a patient diagnosed with a cervical lesion, to assess the state of HPV infection and potential progression toward full malignancy, thereby assisting in the management and follow-up treatment of such a patient. Currently, there are no good methods to assist in the management of patients with low-grade cervical lesions, where such lesions can either progress or regress. Currently available methods generally test the likelihood of progressing towards malignancy or regressing based on histological grading, which classifies intraepithelial cervical neoplasia lesions into CIN1, CIN2, CIN3 and invasive cancer (Schiffman et al., Lancet 370:890-907 (2007)). A classification of CIN2 or worse (CIN2+) is considered to be the threshold for aggressive surgical procedures. However, CIN2 diagnosis by histology alone is highly variable and imprecise, leading to both under- and over-treatment (Stoler and Schiffman, J. Am. Med. Assoc. 285:1500-1505 (2001)). The inability to predict which women will progress to higher-grade lesions from those women who will have regression results in all women being given the same intensive follow-up and testing, which wastes financial and medical resources and also reduces the patient's work productivity.

The HPV E6/E7 nucleic acid-based methods of the invention disclosed herein can better define the HPV infection status and oncogenic activity of the cervical lesion and better reflect disease progression or regression (Ho et al. Int. J. Cancer 127, 622-632 (2010)). Therefore, an embodiment of the present invention is to use an ISH assay to detect the spatial pattern and levels of E6 and/or E7 mRNA and/or DNA of high-risk HPV subtypes. One group of such subtypes is the HPV subgroup of 18, as disclosed herein. Another group of such subtypes is the HPV subgroup of 15, as disclosed herein. HPV-16 alone can also be used as a high-risk HPV subtype. By determining the HPV infection state of a cervical sample, including a sample containing a predetermined lesion such as a CIN grade in a patient, the present invention provides a basis to more knowledgeably triage patients based on the nature of any HPV infection state in the cervical tissue sample or lesion. This provides the clinician with more information on the risk of disease progression and can be used to more efficiently manage individual patients with respect to possible multiple office visits and/or repeated cytology and colposcopy examinations. By better assessing the stage of disease progression and risk for viral persistence and potential cancer development, the methods disclosed herein providing an ISH HPV nucleic acid assay can thus provide objective information for optimal disease management. For example, based on the assessment of the stage of disease progression and risk for cancer development, one can select treatment options that are best for the patient. One can also optimize the frequency for repeating tests for the patient.

As disclosed herein, the invention relates to methods of categorizing, classifying and/or stratifying a cervical tissue sample based on sensitive detection of high risk HPV subtypes using an *in situ* hybridization (ISH) assay (see Examples I and II). The assay is based on the ability to simultaneously detect HPV DNA and mRNA in the same sample, thereby providing information on the HPV infection state of the cervical tissue sample. As disclosed herein, information on the HPV infection state can be used to complement histological analysis of cervical samples to provide a more effective diagnosis than histological analysis or previous HPV testing alone. The methods of the invention can be used to categorize a cervical tissue sample, to confirm a histological analysis of a cervical tissue sample, or to recategorize a cervical tissue sample previously categorized by histology and/or the presence of HPV.

In one embodiment, the invention provides a method of categorizing a cervical tissue sample. The method can comprise performing an *in situ* hybridization assay using an antisense E6 and/or E7 probe on a cervical tissue sample, wherein the antisense E6 and/or E7 probe can simultaneously detect high-risk HPV DNA and HPV RNA; detecting the presence of HPV nucleic acid; and categorizing the cervical tissue sample, wherein the presence of diffuse nuclear staining of HPV nucleic acid only in the superficial layer of the epithelium of the cervical sample indicates a transient state, wherein the presence of granular staining of HPV nucleic acid in the cytoplasm and/or the nucleus in the intermediate and basal layers of the epithelium of the cervical sample indicates a persistent state, or wherein the presence of diffuse nuclear staining of HPV nucleic acid in the superficial layer of the epithelium and granular staining of HPV nucleic acid in the cytoplasm and/or the nucleus of the intermediate layer of the epithelium of the cervical sample indicates a transition state.

The methods of the invention can be used to categorize a cervical tissue sample. As disclosed herein, it was expected that high risk HPV subtypes would exhibit upregulation of E6/E7 mRNA expression in high grade CIN lesions (CIN2+) of cervical samples. It was surprisingly found that HPV nucleic acids were detected in low grade cervical lesions previously categorized as CIN1 (see Example II). HPV nucleic acids were detected in all three categories of cervical lesions, CIN1, CIN2 and CIN3. However, the pattern of detection of HPV nucleic acid in each category of CIN lesions was distinct for each category (see Example II). It was further discovered that antisense probes directed to the E6 and/or E7 region of high risk HPV subtypes could simultaneously detect both HPV DNA and HPV mRNA, and the simultaneous detection of HPV DNA and mRNA allows further categorization of the cervical tissue sample based on the HPV infection state of cells of the cervical sample.

In particular and as disclosed herein, it was determined that cervical tissue samples previously categorized as CIN1 exhibited diffuse nuclear staining of HPV nucleic acid but only in the superficial layer of the epithelium of the cervical sample. It was demonstrated that the HPV nucleic acid present in the CIN1 sample is HPV DNA. A cervical tissue sample previously categorized as CIN3 also exhibited a distinct HPV nucleic acid staining pattern. In the CIN3 lesion, the cervical sample exhibited granular staining of HPV nucleic acid in the cytoplasm and the nucleus throughout the epithelium, most prounounced in the intermediate and basal layers of the cervical epithelium. A cervical tissue sample previously categorized as CIN2 had yet another staining pattern, which appeared to be a hybrid between the pattern of the CIN1 and CIN3 cervical lesions. In particular in the CIN2 lesion, the cervical sample exhibited diffuse nuclear staining of HPV nucleic acid in the superficial layer as well as granular staining of HPV nucleic acid in the cytoplasm and the nucleus of cells of the intermediate layer of the cervical epithelium. The diffuse nuclear staining of HPV nucleic acid has a similar appearance to that of the CIN1 lesion, whereas the granular staining of HPV nucleic acid in the cytoplasm and nucleus has a similar appearance to that of the CIN3 lesion. Therefore, the HPV nucleic acid staining pattern in a cervical tissue sample can be used to categorize the cervical sample as a low-grade, CIN1-like lesion or as a high-grade, CIN2- or CIN3-like lesion. As described herein and known in the art, a CIN2 lesion is generally categorized as a high-grade lesion.

Further as disclosed herein, the distinct HPV nucleic acid staining patterns of diffuse nuclear staining versus granular cytoplasmic and nuclear staining were determined to correlate with DNA versus mRNA expression. In particular, it was found that the diffuse nuclear staining pattern observed with HPV antisense probes was also observed with HPV sense probes (see Example II). As disclosed herein, a sense probe can hybridize with DNA but not mRNA. This observation was confirmed using RNase, which did not reduce the signal of the diffuse nuclear staining, and with DNase, which reduced the signal. Therefore, it was determined that the diffuse nuclear staining of HPV nucleic acid observed in CIN1 and CIN2 lesions corresponds to HPV DNA.

The observation of diffuse nuclear staining only in the superficial layer of CIN1 lesions that corresponds to HPV DNA is likely viral DNA produced in the productive phase of the viral life cycle, after HPV genome amplification but before the viral capsids are released from the host cells. Although the assay stains each copy of a target nucleic acid as a signal "dot," since HPV genome amplification typically produces very large number of HPV DNA copies, the dotted stain of each copy overlaps with each other to produce the diffuse staining pattern. It is known that CIN1 lesions frequently clear the HPV infection spontaneously. Therefore, the HPV infection state of a CIN1 lesion is transient. The methods of the invention can be utilized to categorize a cervical tissue sample as a transient state that corresponds to a transient HPV infection state.

Additionally as disclosed herein, the granular staining in the nucleus and cytoplasm was found to correspond to HPV mRNA. This was based on the observation that the granular nuclear and cytoplasmic staining pattern was seen with the antisense probe but not the sense probe. This observation was confirmed with RNase treatment, which significantly reduced the granular staining (see Example II). The observation of granular HPV nucleic acid staining in the cytoplasm and/or the nucleus of cells throughout the epithelial layer (most pronounced in the intermediate and/or basal layers) in CIN3 lesions correlates with the establishment of a persistent HPV infection reflected in active E6 and/or E7 mRNA expression. The granular cytoplasmic and nuclear staining of HPV mRNA likely reflects the establishment of a persistent HPV infection in CIN3 lesions. Therefore, the HPV infection state of a CIN3 lesion is persistent. The methods of the invention can be utilized to categorize a cervical tissue sample as a persistent state that corresponds to a persistent HPV infection state.

Further as disclosed herein, a hybrid HPV nucleic acid staining pattern was observed in a CIN2 lesion. As described herein, the diffuse nuclear staining in CIN2, similar to the staining pattern observed in CIN1, was determined to be HPV DNA (see Example II). The granular staining pattern in the cytoplasm and nucleus in the CIN2 lesion corresponds to HPV mRNA expression, similar to the pattern observed in the CIN3 lesion (see Example II). The observation of a hybrid staining pattern in the CIN2 lesion is likely to correlate with a transition HPV infection state, where the HPV infection is transitioning from a transient infection that can self-clear to a persistent HPV infection as reflected in the CIN3 lesion. The methods of the invention can be utilized to categorize a cervical tissue sample as a transition state that corresponds to a transition HPV infection state that has an increased likelihood of transitioning to a persistent HPV infection state. This is particularly useful since CIN2 lesions are the most difficult to categorize by histology alone. By identifying and categorizing a CIN2 lesion based on HPV infection state, the methods of the invention can be used to objectively identify and stratify cervical samples based on the likelihood that the cervical sample exhibits a transition HPV infection state that has an increased likelihood of transitioning to a persistent HPV infection state. Thus, the methods of the invention are particularly useful for stratifying a CIN2 lesion into a cervical sample that is more like a CIN1 state (that is, exhibiting the histology of a CIN2 lesion but the HPV nucleic acid staining pattern of a transient HPV infection state) or a CIN2 lesion that is more likely to progress to the persistent state of a CIN3 lesion based on the detection of active transcription (the presence of mRNA) of E6 and/or E7 of a high risk HPV subtype.

As disclosed herein, the methods of the invention relate to categorizing cervical samples based on the HPV infection state of cervical cells. Based on the infection state of the cervical cells, the methods of the invention can be used to categorize the cervical tissue sample by detection of HPV DNA only in the superficial layer of the epithelium of the cervical tissue sample, which indicates a low-grade lesion. As described herein, the detection of abundant HPV DNA only in the superficial layer of the epithelium of a cervical tissue sample correlates with a transient HPV infection state and a CIN1 lesion. Such a transient HPV infection state or CIN1-like lesion is considered to be a low-grade lesion, as disclosed herein.

In another embodiment, the methods of the invention can be used to categorize the cervical tissue sample by detection of HPV RNA in the superficial, intermediate and/or basal layers of the epithelium of the cervical tissue sample, in particular in the intermediate and/or basal layers, which indicates a high-grade lesion. As disclosed herein, the detection of HPV RNA (mRNA) throughout the layers of the cervical epithelium correlates with a persistent HPV infection state and a CIN3 lesion. Such a persistent HPV infection and CIN3-like lesion is considered to be a high-grade lesion, as disclosed herein.

In still another embodiment, the methods of the invention can be used to categorize the cervical tissue sample by detection of HPV RNA in the basal or intermediate layer and HPV DNA in the superficial layer of the epithelium of the cervical tissue sample, which indicates a high grade lesion. As disclosed herein, the detection of HPV RNA in the intermediate layer and HPV DNA in the superficial layer of the epithelium of a cervical tissue sample correlates with a transition HPV infection state and a CIN2 lesion. While such a transition HPV infection state could be considered as an intermediate state (hybrid state) between CIN1 and CIN3, it is known in the art that a CIN2 lesion has an increased likelihood of transitioning to a CIN3 grade and is routinely assigned as a high-grade lesion (see Table 1 and references such as Sellors and Sankaranarayanan, *supra,* 2003; Kalaf and Cooper, *supra,* 2007; Brown and Trimble, *supra,* 2012; Waxman et al., *supra,* 2012). Thus, even though detection of HPV RNA in the intermediate layer and HPV DNA in the superficial layer of the epithelium of a cervical tissue sample correlates with a transition HPV infection state, this hybrid or intermediate state is still considered as a high-grade lesion for consistency with the known status of a CIN2 lesion and with the presence of active HPV E6/E7 mRNA expression.

As disclosed herein, HPV RNA (mRNA) was detected in both CIN2 and CIN3 lesions, indicating that these lesions are actively transcribing E6 and/or E7 mRNA. In contrast, HPV DNA only, or predominantly DNA, was detected in CIN1 lesions. Therefore, the presence of HPV RNA in cervical tissue samples correlates with CIN2 and CIN3 lesions, both of which are classified as high-grade lesions. Therefore, the methods of the invention can be used to categorize or classify a cervical tissue sample based on the detection of HPV RNA (mRNA) as indicating a high grade lesion.

As disclosed herein, the methods of the invention advantageously permit the simultaneous detection of HPV DNA and HPV RNA. Previous methods for detecting HPV in a cervical sample were not directed to detecting HPV DNA and HPV RNA simultaneously in the same sample using the same detection method. If HPV DNA and HPV RNA were to be detected in the same sample, generally different detection methods were employed to optimize detection of DNA or RNA. This was generally considered as important since detection of even low copy number HPV DNA could be relevant to understanding the pathology of a cervical tissue sample lesion. The methods of the present invention can be used advantageously to detect HPV DNA and HPV RNA simultaneously, at high sensitivity, thereby permitting the classification of cervical tissue samples as disclosed herein and also increase the sensitivity of HPV detection. Therefore, an embodiment of the invention includes the ability to detect both HPV DNA and HPV RNA simultaneously, when both are present in a cervical tissue sample. Thus, detecting the presence of HPV nucleic acid can comprise detecting HPV DNA and HPV RNA. In a particular embodiment, the antisense E6 and/or E7 probe can detect high risk HPV in the probe set selected from one or more of the HPV subtypes 16, 18, 26, 31, 33, 35, 39, 45, 51, 52, 53, 56, 58, 59, 66, 68, 73 and 82.

Further as disclosed herein, the methods of the invention, which can simultaneously detect both HPV DNA and HPV RNA, can include a pretreatment step of the sample to denature double stranded DNA prior to performing the *in situ* hybridization assay. This step can be used to increase or optimize the detection of HPV DNA by generating at least partially denatured, single stranded stretches of DNA that can more efficiently hybridize to the HPV target probes. Methods for treating the cervical cytology sample to at least partially denature the HPV DNA are well known in the art, as disclosed herein. It is understood that such methods can result in denaturation of substantially all of the DNA present in the sample or partially denature the DNA present in the sample so as to increase the efficiency of binding of a target probe to the HPV DNA. One skilled in the art can readily determine appropriate conditions for at least partial denaturation to substantially complete denaturation of DNA in the sample sufficient to provide detection of HPV DNA.

As described herein, the methods of the invention utilize antisense probes to simultaneously detect HPV DNA and HPV RNA, if present. A method of the invention can optionally include hybridizing parallel samples using a sense probe to identify and/or confirm detection of HPV DNA (see Examples I and II). Thus, a parallel assay using sense probes can optionally be employed in methods of the invention. In such a case, the sense probe will generally be a set of target probes to the same HPV subtype or pool of HPV subtypes as used in the antisense probe. The methods of the invention can optionally further include incubation of the sample with DNase and/or RNase to confirm the determination of the detected HPV nucleic acid being HPV DNA or HPV RNA, if desired. Furthermore, it is understood that the level of HPV DNA and/or RNA can be quantified using well known assay conditions for quantifying detected signals in an *in situ* hybridization assay. Thus, the methods of the invention can utilize the HPV DNA and/or RNA expression patterns, the spatial orientation of HPV DNA or RNA expression in tissue samples, or level of HPV DNA and/or RNA expression, or a combination thereof, to determine relevant information on the HPV infection state of the cervical sample.

As disclosed herein, the methods of the invention provide the ability to categorize a cervical tissue sample based on the detection of HPV DNA and/or mRNA, if present, and additionally utilize the spatial expression pattern of HPV DNA and/or mRNA to further categorize the cervical tissue sample. The HPV DNA and/or mRNA expression pattern has been observed in different layers of the cervical epithelium depending on the CIN category of a cervical lesion, in particular expression in the superficial, intermediate and/or basal layer of the epithelium of cervical tissue, for example, expression in the intermediate and/or basal layers of the epithelium. One skilled in the art will readily understand the meaning of the layers of the epithelium of the cervical tissue based on the well known anatomy and histology of cervical tissue.

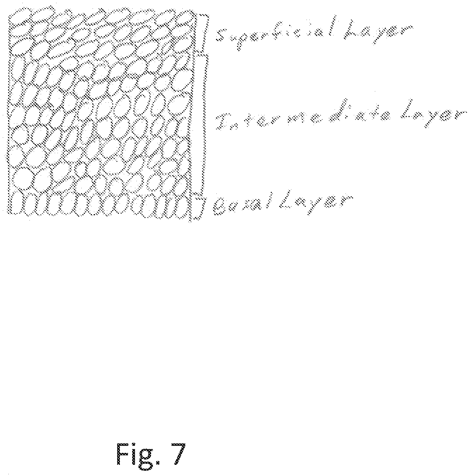

By way of illustration, Figure 7 shows a schematic diagram of the known structure of the normal cervical epithelium. The superficial layer is composed of several layers of loosely attached cells that are generally broader and thinner than those of the intermediate layer. The intermediate layer, also referred to as the mid or parabasal zone, is composed of maturing squamous cells, which have more abundant cytoplasm than those of the basal layer. Sometimes the intermediate layer is further stratified into a parabasal layer and more mature squamous epithelial cells. As used herein, term "intermediate layer" includes parabasal and the more mature squamous epithelial cells, that is, the layer between the basal layer and the superficial layer. The basal layer, also referred to as the basal zone, is a single layer of cylindrical cells, which have the main function of epithelial regeneration. Figure 7 is intended to illustrate the general location of the superficial, intermediate and basal layers of the epithelium of cervical tissue, and one skilled in the art, in particular a clinician or pathologist, will readily understand the meaning of the superficial, intermediate and basal layers of the epithelium of cervical tissue as used herein.

As described herein, the methods of the invention can be used to determine helpful information on HPV infection status of a cervical tissue sample and categorization based on the spatial orientation of the expression pattern of high risk HPV DNA and/or mRNA, in particular as determined in particular layers of the cervical epithelium. The methods of the invention can also be used to categorize a cervical cytology sample, even in the absence of spatial information on the expression pattern of HPV DNA and/or mRNA. As disclosed herein, Caski cells were used as a model to characterize the expression of HPV DNA and mRNA in individual cells in the absence of a tissue structure such as a tissue biopsy. Caski cells are a cervical cell line known to harbor HPV16. As disclosed herein, HPV-16 sense and antisense probes could be used to detect HPV DNA and mRNA in individual cells. The detection of HPV DNA and mRNA in individual cells indicates that the methods of the invention can readily be applied to cytological samples such as cervical cells obtained from cervical cell scrapings, for example, as obtained with a Pap smear.

In an additional embodiment, the invention provides a method of categorizing a cervical cytology sample. The sample can be obtained in routine a cervical examination procedure such as a Pap smear or similar technique. The collected cervical cells can be directly spread onto microscope slides at the point of collection or collected as suspended cells in preservative buffer (that is, liquid Pap) and later deposited onto slides using methods known in the art. The method can comprise performing an *in situ* hybridization assay using an antisense E6 and/or E7 probe on a cervical cytology sample, wherein the antisense E6 and/or E7 probe can simultaneously detect HPV DNA and HPV RNA; detecting the presence of HPV nucleic acid; and categorizing the cervical cytology sample, wherein the presence of diffuse nuclear staining of HPV nucleic acid only in the cervical cell sample indicates a transient state, wherein the presence of granular staining of HPV nucleic acid in the cytoplasm and the nucleus in the cervical cytology sample indicates a persistent state, or wherein the presence of diffuse nuclear staining of HPV nucleic and granular staining of HPV nucleic acid in the cytoplasm and the nucleus of the cervical cytology sample indicates a transition state.

Similar to the methods using a cervical tissue sample, the methods of categorizing a cervical cytology sample can be used to determine a transient state that corresponds to a transient HPV infection state. As disclosed herein, cells of a CIN1 lesion exhibited staining of HPV DNA only. Therefore, a cervical cytology sample such as a cervical cell scraping that contains cervical cells exhibiting staining of HPV DNA only, and in particular diffuse nuclear staining, are considered to correspond to a transient HPV infection and a low-grade lesion (CIN1-like lesion).

In another embodiment of a method of categorizing a cervical cytology sample, the detection of HPV mRNA expression indicates a high-grade lesion. In the case where granular nuclear and cytoplasmic HPV nucleic acid staining is observed, such a sample is considered to represent the expression of HPV mRNA and to correspond to a high-grade lesion. In such a sample, where cells in a representative cytological specimen exhibit nuclear and cytoplasmic mRNA expression, the sample is considered to correlate with a high-grade, CIN3-like lesion. A sample where cells in a representative cytological specimen exhibit nuclear and cytoplasmic mRNA expression is considered as a persistent state that corresponds to a persistent HPV infection state.

In still another embodiment of a method of categorizing a cervical cytology sample, the detection of both granular nuclear and cytoplasmic HPV nucleic acid staining and diffuse nuclear staining in cells within the same cytological sample corresponds to a transition state, similar to the CIN2 state observed by cervical tissue analysis, corresponds to an HPV infection state that has an increased likelihood of transitioning to a persistent HPV infection state. In a method of the invention, detection of HPV RNA observed in the cervical cytology sample indicates a high-grade lesion. Similar to the cervical tissue sample analysis, expression of HPV E6 and/or E7 mRNA is an indication of active transcription of HPV E6 and/or E7 and corresponds to a high grade lesion. Detection of HPV RNA and HPV DNA in the cervical cytology sample also indicates a high grade lesion. In addition to detection of HPV mRNA and active transcription, the detection of cells in a representative cytological specimen that also contain HPV DNA can correspond to a CIN2-like lesion.

It is understood that the description herein relating to cervical tissue samples can similarly be applied to a cervical cytology sample, as appropriate. Accordingly, in a method of categorizing a cervical cytology sample, detecting the presence of HPV nucleic acid can comprise detecting HPV DNA and HPV RNA. In addition, in a method of categorizing a cervical cytology sample, the sample can be pretreated to denature double stranded DNA prior to performing the *in situ* hybridization assay, as described herein.

In another embodiment, the invention provides a method of categorizing a cervical cytology sample. The method can comprise performing an *in situ* hybridization assay using an antisense E6 or E7 probe on a cervical cytology sample, wherein the antisense E6 or E7 probe can simultaneously detect high-risk HPV DNA and HPV RNA; detecting the presence of HPV nucleic acid; and categorizing the cervical cytology sample, wherein the presence of diffuse nuclear staining of HPV nucleic acid only in a cell of the cervical cytology sample indicates a transient HPV infection state in the cell, or wherein the presence of granular staining of HPV nucleic acid in the cytoplasm and/or the nucleus in a cell of the cervical cytology sample indicates a persistent HPV infection state in the cell. In such a method of the invention, presence of only diffuse nuclear staining of HPV nucleic acid in a plurality of cells of the cervical cytology sample, for example, HPV positive cells in the sample such as all or substantially all of the HPV positive cells, indicates the sample corresponds to a transient HPV infection state. In another embodiment, the presence of granular staining of HPV nucleic acid in the cytoplasm and/or nucleus in a plurality of cells of the cervical cytology sample, for example, HPV positive cells in the sample such as all or substantially all of the HPV positive cells, indicates the sample corresponds to a persistent HPV infection state. In still another embodiment, the presence of diffuse nuclear staining of HPV nucleic acid and granular staining of HPV nucleic acid in the cytoplasm and/or nucleus in a plurality of cells of the cervical cytology sample, for example, HPV positive cells in the sample such as all or substantially all of the HPV positive cells, indicates the sample corresponds to a transition HPV infection state. In another embodiment, the presence of a mixture of HPV positive cells having granular staining of HPV nucleic acid in the cytoplasm and/or nucleus and cells having diffuse nuclear staining of HPV nucleic acid indicates a likelihood of transitioning to a persistent HPV infection state. In another embodiment of a method of the invention, the presence of an increasing ratio, relative ratio or a greater number of cells having granular staining of HPV nucleic acid in the cytoplasm and/or nucleus relative to cells having diffuse nuclear staining of HPV nucleic acid indicates an increased likelihood of transitioning to a persistent HPV infection state.

In the methods of the invention, t presence of HPV nucleic acid comprises detecting HPV DNA and HPV RNA. In another embodiment of the methods of the invention, the antisense E6 and/or E7 probe can detect high risk HPV selected from one or more of the HPV subtypes 16, 18, 26, 31, 33, 35, 39, 45, 51, 52, 53, 56, 58, 59, 66, 68, 73 and 82. In still another embodiment of the method of the invention, the sample is pretreated to denature double stranded DNA prior to performing the *in situ* hybridization assay.

As used herein, a cervical tissue sample is considered to relate to a tissue sample obtained from an individual such as by tissue biopsy. In the case of a tissue sample, the anatomy of the tissue sample is preserved to some extent for the assay, for example, by using a formalin fixed and paraffin embedded (FFPE) tissue sample, a tissue slice, and so forth as understood by those skilled in the art to preserve the relative orientation of certain cells within the tissue sample. As used herein, a cervical cytology sample refers to a cytological sample where the orientation of cells in a tissue is not necessarily preserved. Such a cytological sample of a cervical tissue can be obtained, for example, using a Pap smear or similar technique used to obtain representative cervical cells from an individual. Alternatively, such a cytological sample can be obtained from a tissue biopsy, where cells from the tissue are dispersed in the tissue using well known methods such as by mincing the tissue sample or using other methods of mechanical disruption or enzymatic digestion such as with proteases (see, for example, Freshney, Culture of Animal Cells: A Manual of Basic Technique 4th ed., Wiley-Liss, New York (2000)). In such a case, the relative orientation of cells in the tissue sample is not preserved in the cytological sample. One skilled in the art will readily understand the meaning of a tissue sample and a cytology sample, as disclosed herein.

As described herein, the methods of the invention can be used to categorize cervical tissue or cytology samples based on detection of HPV DNA and mRNA, in particular E6 and/or E7 of high-risk HPV subtypes. The methods can be employed on cervical tissue or cytology samples which have not been previously analyzed by histology and assigned as a category based on HPV infection state alone. Alternatively, the methods of the invention can be used on samples used for histological analysis to provide a confirmatory diagnosis of low-grade or high-grade lesion, as disclosed herein. Furthermore, the methods of the invention can be used on samples, either tissue biopsies or cytological samples, that have been previously categorized by traditional histological analysis. In the case where the HPV infection state corresponds to the previously determined grade, such as low-grade or high-grade lesion or CIN1, CIN2 or CIN3 category based on the analysis disclosed herein, the methods of the invention can be used as a confirmatory assay for the histological categorization. If the HPV infection state determined by the methods of the invention are different than the previously determined histological analysis, the methods of the invention can be used to recategorize or reclassify a previously categorized cervical tissue or cytological sample. Thus, the methods of the invention can be used alone as a method to analyze cervical tissue or cytological samples or in combination with traditional histological analysis to either confirm or recategorize a grade determined by histology.

In addition, the methods of the invention can also be used to assess the disease state or likelihood of progressing to a disease state (that is, prognosis). As disclosed herein, the methods of the invention can be employed to categorize a cervical tissue or cytology sample as one exhibiting a transient HPV infection state and therefore to be more likely to regress and spontaneously clear as a cervical infection. In addition, the methods of the invention can be employed to categorize a cervical tissue or cytology sample as one exhibiting a persistent high risk HPV infection and therefore considered to be more likely to progress to a cancerous state. The methods of the invention can therefore be used to determine the risk of progression to a neoplastic state. Further, the methods of the invention can be employed to categorize a cervical tissue or cytology sample as one exhibiting a transition HPV infection state. Such a state is one that is more likely to progress to a higher grade such as CIN3. Such methods are particularly useful for categorizing CIN2 lesions as a lesion more likely to regress to a CIN1 grade and spontaneously clear or more likely to progress to a CIN3 grade with increased likelihood to progress to a cancerous stage, thereby providing improved treatment or non-treatment regimens.

In another embodiment, the invention provides a method of determining the prognosis of patient outcome based on analysis of a cervical tissue sample. The method can comprise performing an *in situ* hybridization assay using an antisense E6 or E7 probe on a cervical tissue sample, wherein the antisense E6 or E7 probe can simultaneously detect high-risk HPV DNA and HPV RNA; detecting the presence of HPV nucleic acid; and categorizing the cervical tissue sample, wherein the presence of diffuse nuclear staining of HPV nucleic acid only in the superficial layer of the epithelium of the cervical sample indicates a transient HPV infection state, wherein the presence of granular staining of HPV nucleic acid in the cytoplasm and/or the nucleus in the intermediate and basal layers of the epithelium of the cervical sample indicates a persistent HPV infection state, or wherein the presence of diffuse nuclear staining of HPV nucleic acid in the superficial layer of the epithelium and granular staining of HPV nucleic acid in the cytoplasm and/or the nucleus of the intermediate layer of the epithelium of the cervical sample indicates a transition HPV infection state.

In a particular embodiment of such a method of the invention, determination of a transient HPV infection state indicates an increased likelihood of clearing the HPV infection. In another embodiment, determination of a persistent infection state indicates an increased likelihood of progressing to a cancerous state. In yet another embodiment, determination of a transition HPV infection state indicates an increased likelihood of progressing to a persistent HPV infection state. In another embodiment, determination of a transition HPV infection state indicates an increased likelihood of progressing to a cancerous state. In still another embodiment, detecting the presence of HPV nucleic acid comprises detecting HPV DNA and HPV RNA. In yet another embodiment, the high risk HPV in the probe set used in the method of the invention is selected from one or more of the HPV subtypes 16, 18, 26, 31, 33, 35, 39, 45, 51, 52, 53, 56, 58, 59, 66, 68, 73 and 82, for example, the antisense E6 and/or E7 probe can detect high risk HPV selected from one or more of the HPV subtypes 16, 18, 26, 31, 33, 35, 39, 45, 51, 52, 53, 56, 58, 59, 66, 68, 73 and 82. In another embodiment of a method of the invention, the sample can be pretreated to denature double stranded DNA prior to performing the *in situ* hybridization assay.

In still another embodiment, the invention provides a method of determining the prognosis of patient outcome based on analysis of a cervical cytology sample. Such a method can comprise performing an *in situ* hybridization assay using an antisense E6 or E7 probe on a cervical cytology sample, wherein the antisense E6 or E7 probe can simultaneously detect high-risk HPV DNA and HPV RNA; detecting the presence of HPV nucleic acid; and categorizing the cervical cytology sample, wherein the presence of diffuse nuclear staining of HPV nucleic acid only in a cell of the cervical cytology sample indicates a transient HPV infection state in the cell, or wherein the presence of granular staining of HPV nucleic acid in the cytoplasm and/or the nucleus in a cell of the cervical cytology sample indicates a persistent HPV infection state in the cell.

In a particular embodiment of such a method of the invention, the presence of only diffuse nuclear staining of HPV nucleic acid in a plurality of cells of the cervical cytology sample, for example, HPV positive cells in the sample such as all or substantially all of the HPV positive cells, indicates the sample corresponds to a transient HPV infection state. In another embodiment of a method of the invention, the presence of granular staining of HPV nucleic acid in the cytoplasm and/or nucleus in a plurality of cells of the cervical cytology sample, for example, HPV positive cells in the sample such as all or substantially all of the HPV positive cells, indicates the sample corresponds to a persistent HPV infection state. In still another embodiment, the presence of diffuse nuclear staining of HPV nucleic acid and granular staining of HPV nucleic acid in the cytoplasm and/or nucleus in a plurality of cells of the cervical cytology sample, for example, HPV positive cells in the sample such as all or substantially all of the HPV positive cells, indicates the sample corresponds to a transition HPV infection state. In another embodiment, the presence of a mixture of HPV positive cells having granular staining of HPV nucleic acid in the cytoplasm and/or nucleus and cells having diffuse nuclear staining of HPV nucleic acid indicates a likelihood of transitioning to a persistent HPV infection state. In yet another embodiment, the presence of a an increasing ratio, relative ratio or a greater number of cells having granular staining of HPV nucleic acid in the cytoplasm and/or nucleus relative to cells having diffuse nuclear staining of HPV nucleic acid indicates an increased likelihood of transitioning to a persistent HPV infection state.

In still another embodiment of a method of the invention, determination of a transient HPV infection state indicates an increased likelihood of clearing the HPV infection. In another embodiment, determination of a persistent infection state indicates an increased likelihood of progressing to a cancerous state. In yet another embodiment, determination of a transition HPV infection state indicates an increased likelihood of progressing to a persistent HPV infection state. In another embodiment, determination of a transition HPV infection state indicates an increased likelihood of progressing to a cancerous state. In still another embodiment of a method of the invention, detecting the presence of HPV nucleic acid can comprise detecting HPV DNA and HPV RNA. In yet another embodiment of a method of the invention, the high risk HPV in the probe is selected from one or more of the HPV subtypes 16, 18, 26, 31, 33, 35, 39, 45, 51, 52, 53, 56, 58, 59, 66, 68, 73 and 82, for example, the antisense E6 and/or E7 probe can detect high risk HPV selected from one or more of the HPV subtypes 16, 18, 26, 31, 33, 35, 39, 45, 51, 52, 53, 56, 58, 59, 66, 68, 73 and 82. In another embodiment, the sample can be pretreated to denature double stranded DNA prior to performing the in situ hybridization assay.

As is well known in the art, appropriate negative controls can optionally be included in a hybridization assay, as disclosed herein. For example, a hybridization assay can be conducted using at least one negative control probe set. The use and design of negative controls for use in an *in situ* hybridization assay are well known to those skilled in the art. Particularly useful negative controls include those that would not hybridize to the particular HPV subtype(s) in the target probe set being characterized. Such a negative control can therefore be based on probes that hybridize to sequences known not to be expressed in a cervical cell, for example, tissue specific probes for other cell types. A particularly useful negative control would be a probe that is designed for a completely different organism, in particular a non-mammalian organism, and more particularly a non-eukaryotic organism such as a bacterium. One skilled in the art will readily know how to select appropriate negative controls suitable for an *in situ* hybridization assay.

As disclosed herein, one method for performing *in situ* hybridization based assays of the invention include the use of an RNAscope® assay. RNAscope® *in situ* hybridization assays are commercially available from Advanced Cell Diagnostics, Inc. (Hayward CA). RNAscope® assays have been described previously, for example, in U.S. Patent No. 7,709,198, U.S. publications 2008/0038725 and 2009/0081688, and WO 2007/001986 and WO 2007/002006. RNAscope® assays in various embodiments are described below in more detail. It is understood that the assays of the invention can be performed with commercially available *in situ* hybridization assays, including RNAscope® or variations of such assays as described herein. The RNA ISH technology platform called RNAscope® has single-molecule sensitivity, is capable of multiplex detection, and is compatible with formalin fixed and paraffin embedded (FFPE) tissue (see, for example, Masand et al., 5:108-116 (2011); Ukpo et al., Am. J. Surg. Pathol. 35:1343 (2011); Wang et al., J. Mol. Diagnostics 14:22-29 (2012)). The use of RNAscope® for detecting HPV has also been described (see WO 2012/103414 and US publication 20120214152). A summary and more details of an RNAscope® assay are described herein.

As used herein, the term "label probe" refers to an entity that binds to a target molecule, directly or indirectly, and allows the target to be detected. A label probe (or "LP") contains a nucleic acid binding portion that is typically a single-stranded polynucleotide or oligonucleotide that comprises one or more labels which directly or indirectly provides a detectable signal. The label can be covalently attached to the polynucleotide, or the polynucleotide can be configured to bind to the label. For example, a biotinylated polynucleotide can bind a streptavidin-associated label. The label probe can, for example, hybridize directly to a target nucleic acid, such as a high risk HPV subtype, or it can hybridize to a nucleic acid that is in turn hybridized to the target nucleic acid or to one or more other nucleic acids that are hybridized to the target nucleic acid. Thus, the label probe can comprise a polynucleotide sequence that is complementary to a polynucleotide sequence, particularly a portion, of the target nucleic acid. Alternatively, the label probe can comprise at least one polynucleotide sequence that is complementary to a polynucleotide sequence in an amplifier, preamplifier, intermediary complex, or the like, as described herein. As used herein, a label probe comprising an enzyme label or non-enzyme label refers to a label probe comprising a nucleic acid binding portion such as an oligonucleotide and an enzyme or non-enzyme label that is coupled to the nucleic acid binding portion. As disclosed herein, the coupling of the enzyme or non-enzyme label to the nucleic acid binding portion can be covalent or through a high affinity binding interaction such as biotin/avidin or other similar high affinity binding molecules.

As used herein, a "target probe" is a polynucleotide that is capable of hybridizing to a target nucleic acid and capturing or binding a label probe or intermediary complex molecule to that target nucleic acid. The target probe can hybridize directly to the label probe, or it can hybridize to one or more nucleic acids that in turn hybridize to the label probe; for example, the target probe can hybridize to an amplifier or a preamplifier in an intermediary complex. The target probe thus includes a first polynucleotide sequence that is complementary to a polynucleotide sequence of the target nucleic acid and a second polynucleotide sequence that is complementary to a polynucleotide sequence of the label probe, amplifier, preamplifier, or the like. The target probe is generally single-stranded so that the complementary sequence is available to hybridize with a corresponding target nucleic acid, label probe, amplifier or preamplifier.

As used herein, an "antisense probe" refers to a polynucleotide or oligonucleotide that is complementary to mRNA. An antisense probe can therefore hybridize to mRNA. Such an antisense probe can also hybridize to the DNA strand that has the corresponding sequence as the mRNA, also referred to as the coding strand. An antisense probe orientation is also known in the art as corresponding to the noncoding strand. As used herein, a "sense probe" refers to a polynucleotide or oligonucleotide that has the same sequences as the sense strand or coding strand of DNA or the mRNA. A sense probe can therefore hybridize to a complementary noncoding strand of DNA but cannot hybridize to mRNA. Therefore, as used herein, an antisense probe can hybridize to a complementary strand on both DNA and mRNA whereas a sense probe can hybridize to a complementary strand on DNA only.

As used herein, an "amplifier" is a molecule, typically a polynucleotide, that is capable of hybridizing to multiple label probes. Typically, the amplifier hybridizes to multiple identical label probes. The amplifier can also hybridize to a target nucleic acid, at least one target probe or nucleic acid bound to a target probe such as a preamplifier. For example, the amplifier can hybridize to at least one target probe and to a plurality of label probes, or to a preamplifier and a plurality of label probes. The amplifier can be, for example, a linear, forked, comb-like, or branched nucleic acid. As described herein for all polynucleotides, the amplifier can include modified nucleotides and/or nonstandard internucleotide linkages as well as standard deoxyribonucleotides, ribonucleotides, and/or phosphodiester bonds. Suitable amplifiers are described, for example, in U.S. Patent Nos. 5,635,352, 5,124,246, 5,710,264, 5,849,481, and 7,709,198 and U.S. publications 2008/0038725 and 2009/0081688.

As used herein, a "preamplifier" is a molecule, typically a polynucleotide, that serves as an intermediate between one or more target probes and one or more amplifiers. Typically, the preamplifier hybridizes simultaneously to one or more target probes and to a plurality of amplifiers. Exemplary preamplifiers are described, for example, in U.S. Patent Nos. 5,635,352, 5,681,697 and 7,709,198 and U.S. publications 2008/0038725 and 2009/0081688.

As used herein, an "intermediary complex" is a molecule, and can be a large, complex molecule, or an assembly of multiple molecules, that has one or more components, each containing a section capable of binding specifically to a section of the target nucleic acid and has another component or multiple components each containing one or multiple sections capable of binding to the label probe. An intermediary complex can comprise, for example, a label probe, amplifier and/or preamplifier.

In one aspect, the label probes bind directly to the target probes. In another aspect, the label probes are captured to the target probes indirectly, for example, through binding of preamplifiers and/or amplifiers. Use of amplifiers and preamplifiers can be advantageous in increasing signal strength, since they can facilitate binding of large numbers of label probes to each nucleic acid target. In one embodiment, one target probe hybridizes to each label probe, amplifier, or preamplifier. In another embodiment, two or more target probes hybridize to the label probe, amplifier, or preamplifier.

In embodiments in which two or more target probes are employed, the target probes can hybridize to nonoverlapping polynucleotide sequences in their respective nucleic acid target. The target probes can, but need not, cover a contiguous region of the nucleic acid target. Blocking probes, polynucleotides which hybridize to regions of the nucleic acid target not occupied by target probes, can optionally be included and hybridized to the target. For a given nucleic acid target, the corresponding target probes and blocking probes are generally complementary to physically distinct, nonoverlapping sequences in the nucleic acid target, which nonoverlapping sequences can be, but are not necessarily, contiguous. Having the target probes and optional blocking probes be contiguous with each other can in some embodiments enhance hybridization strength, remove secondary structure, and ensure more consistent and reproducible signal.

The ISH methods disclosed herein are particularly useful for multiplex detection of nucleic acids, including simultaneous detection of one or more, two or more, three or more, or more than three nucleic acid targets such as three or more high risk HPV subtypes. Thus, the cell can optionally comprise or be suspected of comprising a second, third, fourth, fifth, sixth, seventh or even more, including up to 18, nucleic acid targets such as high risk HPV subtypes. For example, the method detecting a third nucleic acid target comprises: providing a third label probe comprising a third label, wherein a third signal from the third label can be either distinguishable or indistinguishable from the first two signals depending the degree of information to be collected, providing at least two third target probe, hybridizing in the cell the third target probe to the third nucleic acid target (when the third target is present in the cell), capturing the third label probe to the third target probe, and detecting the third signal from the third label. The same principle can be applied to detect a fourth, fifth, sixth, seventh, and so forth, including up to all 18 high risk HPV subtypes by using a probe set comprising probes that can specifically hybridize to each of the 18 high risk HPV subtypes, or a subset thereof, as disclosed herein. As disclosed herein, the HPV subtypes can be detected with the same label or distinct labels, as desired.

In detection of nucleic acid targets in a cell, the cell is optionally fixed and permeabilized before hybridization of the target probes. Fixing and permeabilizing cells can facilitate retaining the nucleic acid targets in the cell and permit the target probes, label probes, amplifiers, preamplifiers, and so forth, to enter the cell. The cell is optionally washed to remove materials not captured to one of the nucleic acid targets. The cell can be washed after any of various steps, for example, after hybridization of the target probes to the nucleic acid targets to remove unbound target probes, after hybridization of the preamplifiers, amplifiers, and/or label probes to the target probes, and/or the like.

The various capture and hybridization steps can be performed simultaneously or sequentially, in essentially any convenient order. In a particular embodiment, a given hybridization step is accomplished for all of the nucleic acid targets at the same time to increase efficiency and reduce time, reagents and costs for the assay. For example, all the target probes (first, second, etc.) can be added to the cell at once and permitted to hybridize to their corresponding targets, the cell can be washed, amplifiers (first, second, etc.) can be hybridized to the corresponding target probes, the cell can be washed, the label probes (first, second, etc.) can be hybridized to the corresponding amplifiers, and the cell can then be washed again prior to detection of the labels. As another example, the target probes can be hybridized to the targets, the cell can be washed, amplifiers and label probes can be added together and hybridized, and the cell can then be washed prior to detection. It will be evident that double-stranded nucleic acid target(s) are optionally denatured, for example, by heat or other methods, prior to hybridization of the corresponding target probe(s) to the target(s).

In an embodiment of the invention, the tissue or cell sample is pretreated, in particular to facilitate hybridization to HPV DNA, prior to performing the *in situ* hybridization assay. The sample can be pretreated with buffers and/or heat to at least partially denature the double stranded DNA to facilitate hybridization of the target probe(s) to the HPV DNA present in the sample. Conditions for denaturing double stranded DNA are well known in the art (see Sambrook et al., Molecular Cloning: A Laboratory Manual, Third Ed., Cold Spring Harbor Laboratory, New York (2001); Ausubel et al., Current Protocols in Molecular Biology, John Wiley and Sons, Baltimore, MD (1999)). It is well known in the art that the temperature and buffer conditions can be varied to achieve desired conditions for at least partial denaturation of double stranded DNA in the sample. Such conditions include, but are not limited to, heating the sample, for example, heating the sample between 40°C-110°C, for example, heating the sample at 40°C, 45°C, 50°C, 55°C, 60°C, 65°C, 70°C, 75°C, 80°C, 85°C, 86°C, 87°C, 88°C, 89°C, 90°C, 91°C, 92°C, 93°C, 94°C, 95°C, 96°C, 97°C, 98°C, 99°C, 100°C, 101°C, 102°C, 103°C, 104°C, 105°C, 106°C, 107°C, 108°C, 109°C, or 110°C, or in ranges of 50°C-110°C, 60°C-110°C, 70°C-110°C, 80°C-110°C, 90°C-110°C, 90°C-100°C, 100°C-110°C, or even higher temperatures, as desired. One skilled in the art will readily understand that selection of a particular temperature for denaturation of double stranded in a sample can be varied depending on the buffer conditions for the denaturation step, including but not limited to the presence of salts and/or chaotropic agents, and the like. For example, lower incubation temperatures can generally be used in the presence of chemical denaturing reagents, as is well known in the art. Similarly, one skilled in the art can vary the length of time for carrying out the pretreatment step to effect at least partial denaturation of double stranded DNA. For example, the pretreatment step can be carried out for about 1 minute to about 1 hour, or longer, as desired. In particular, the pretreatment step is carried out for at least 5 minutes to 60 minutes, for example, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 minutes, as desired. Longer incubation times can also be used, for example, 1, 1.5, 2, 2.5, 3, 3.5, 4, 4.5, 5, 6, 7, 8, 9, 10, 11, 12, 13, 14, 15, 16 hours, for example, overnight or longer, as desired, in particular when lower temperatures are used. In a particular embodiment, the pretreatment step is carried out at a temperature of about 100°C for 5-60 minutes, in particular, 5 minutes, 10 minutes, 15, minutes, 20 minutes, 25 minutes, 30 minutes, 40 minutes, 45 minutes, 50 minutes, 55 minutes, 60 minutes, for example, 5-55, 5-50, 5-45, 5-40, 5-35, 5-30, 5-25, 5-20, 5-15, 5-10, 10-60, 10-55, 10-50, 10-45, 10-40, 10-35, 10-30, 10-25, 10-20, 10-15, 15-60, 15-55, 15-50, 15-45, 15-40, 15-35, 15-30, 15-25, 15-20, 20-60, 20-55, 20-50, 20-45, 20-40, 20-35, 20-30, 25-60, 25-55, 25-50, 25-45, 25-40, 25-35, 25-30, 30-60, 30-55, 30-50, 30-45, 30-40, 30-35, 35-60, 35-55, 35-50, 35-45, 35-40, 40-60, 40-55, 40-50, 40-45, 50-60, 50-55, 55-60 minutes, and so forth, as desired.

The cervical cytology sample can be used as a cell suspension for all or most of the steps of the method, for ease of handling. In general, the methods are also applicable to, and particularly useful for analyzing, cervical cell samples in solid tissue samples (for example, tissue biopsies or tissue sections) and/or cells of a cytology sample immobilized on a substrate (for example, a slide or other surface).

In an embodiment of the invention, the ISH methods disclosed herein can utilize distinct enzymes or non-enzyme labels that allow the association of a distinguishable detectable label with a particular target nucleic acid. For example, a distinguishable label can be used to detect each HPV subtype in an HPV target probe set. In another embodiment, an indistinguishable label, that is the same label, is used to detect all HPV target probes, thereby detecting any of the HPV subtypes in the sample. Since the probe set contains high risk HPV subtypes, detecting any one or more of the high risk HPV subtypes in the sample is sufficient for the analysis relating to the methods of the invention. Accordingly, in general, the same label probe is used to detect any and all HPV subtypes present in the sample.

Any of a number of enzymes or non-enzyme labels can be utilized so long as the enzymatic activity or non-enzyme label, respectively, can be detected. The enzyme thereby produces a detectable signal, which can be utilized to detect a target nucleic acid. Particularly useful detectable signals are chromogenic or fluorogenic signals. Accordingly, particularly useful enzymes for use as a label include those for which a chromogenic or fluorogenic substrate is available. Such chromogenic or fluorgenic substrates can be converted by enzymatic reaction to a readily detectable chromogenic or fluorescent product, which can be readily detected and/or quantified using microscopy or spectroscopy. Such enzymes are well known to those skilled in the art, including but not limited to, horseradish peroxidase, alkaline phosphatase, β-galactosidase, glucose oxidase, and the like (see Hermanson, Bioconjugate Techniques, Academic Press, San Diego (1996)). Other enzymes that have well known chromogenic or fluoregenic substrates include various peptidases, where chromogenic or fluorogenic peptide substrates can be utilized to detect proteolytic cleavage reactions. The use of chromogenic and fluorogenic substrates is also well known in bacterial diagnostics, including but not limited to the use of α- and β-galactosidase, β-glucuronidase,6-phospho-β-D-galatoside 6-phosphogalactohydrolase, β-gluosidase, α-glucosidase, amylase, neuraminidase, esterases, lipases, and the like (Manafi et al., Microbiol. Rev. 55:335-348 (1991)), and such enzymes with known chromogenic or fluorogenic substrates can readily be adapted for use in methods of the present invention.

Various chromogenic or fluorogenic substrates to produce detectable signals are well known to those skilled in the art and are commercially available. Exemplary substrates that can be utilized to produce a detectable signal include, but are not limited to, 3,3'-diaminobenzidine (DAB), 3,3',5,5'-tetramethylbenzidine (TMB), Chloronaphthol (4-CN)(4-chloro-1-naphthol), 2,2'-azino-bis(3-ethylbenzothiazoline-6-sulphonic acid) (ABTS), o-phenylenediamine dihydrochloride (OPD), and 3-amino-9-ethylcarbazole (AEC) for horseradish peroxidase; 5-bromo-4-chloro-3-indolyl-1-phosphate (BCIP), nitroblue tetrazolium (NBT), Fast Red (Fast Red TR/AS-MX), and p-Nitrophenyl Phosphate (PNPP) for alkaline phosphatase; 1-Methyl-3-indolyl-β-D-galactopyranoside and 2-Methoxy-4-(2-nitrovinyl)phenyl β-D-galactopyranoside for β-galactosidase; 2-Methoxy-4-(2-nitrovinyl)phenyl β-D-glucopyranoside for β-glucosidase; and the like. Exemplary fluorogenic substrates include, but are not limited to, 4-(Trifluoromethyl)umbelliferyl phosphate for alkaline phosphatase; 4-Methylumbelliferyl phosphate bis (2-amino-2-methyl-1,3-propanediol), 4-Methylumbelliferyl phosphate bis (cyclohexylammonium) and 4-Methylumbelliferyl phosphate for phosphatases; QuantaBlu™ and QuantaRed™ for horseradish peroxidase; 4-Methylumbelliferyl β-D-galactopyranoside, Fluorescein di(P-D-galactopyranoside) and Naphthofluorescein di-(P-D-galactopyranoside) for β-galactosidase; 3-Acetylumbelliferyl β-D-glucopyranoside and 4-Methylumbelliferyl-β-D-glucopyranoside for β-glucosidase; and 4-Methylumbelliferyl-α- D-galactopyranoside for α-galactosidase. Exemplary enzymes and substrates for producing a detectable signal are also described, for example, in US publication 2012/0100540. Various detectable enzyme substrates, including chromogenic or fluorogenic substrates, are well known and commercially available (Pierce, Rockford IL; Santa Cruz Biotechnology, Dallas TX; Invitrogen, Carlsbad CA; 42 Life Science; Biocare). Generally, the substrates are converted to products that form precipitates that are deposited at the site of the target nucleic acid. Other exemplary substrates include, but are not limited to, HRP-Green (42 Life Science), Betazoid DAB, Cardassian DAB, Romulin AEC, Bajoran Purple, Vina Green, Deep Space Black™, Warp Red™, Vulcan Fast Red and Ferangi Blue from Biocare (Concord CA; biocare.net/products/detection/chromogens).

Biotin-avidin (or biotin-streptavidin) is a well known signal amplification system based on the fact that the two molecules have extraordinarily high affinity to each other and that one avidin/streptavidin molecule can bind four biotin molecules. Antibodies are widely used for signal amplification in immunohistochemistry and ISH. Tyramide signal amplification (TSA) is based on the deposition of a large number of haptenized tyramide molecules by peroxidase activity. Tyramine is a phenolic compound. In the presence of small amounts of hydrogen peroxide, immobilized Horse Radish Peroxidase (HRP) converts the labeled substrate into a short-lived, extremely reactive intermediate. The activated substrate molecules then very rapidly react with and covalently bind to electron-rich moieties of proteins, such as tyrosine, at or near the site of the peroxidase binding site. In this way, a lot of extra hapten molecules conjugated to tyramide can be introduced at the hybridization site *in situ.* Subsequently, the deposited tyramide-hapten molecules can be visualized directly or indirectly. Such a detection system is described in more detail, for example, in U.S. publication 2012/0100540.

Embodiments described herein can utilize enzymes to generate a detectable signal using appropriate chromogenic or fluorogenic substrates. It is understood that, alternatively, a label probe can have a detectable label directly coupled to the nucleic acid portion of the label probe. Exemplary detectable labels are well known to those skilled in the art, including but not limited to chromogenic or fluorescent labels (see Hermanson, Bioconjugate Techniques, Academic Press, San Diego (1996)). Exemplary fluorophores useful as labels include, but are not limited to, rhodamine derivatives, for example, tetramethylrhodamine, rhodamine B, rhodamine 6G, sulforhodamine B, Texas Red (sulforhodamine 101), rhodamine 110, and derivatives thereof such as tetramethylrhodamine-5-(or 6), lissamine rhodamine B, and the like; 7-nitrobenz-2-oxa-1,3-diazole (NBD); fluorescein and derivatives thereof; napthalenes such as dansyl (5-dimethylaminonapthalene-1-sulfonyl); coumarin derivatives such as 7-amino-4-methylcoumarin-3-acetic acid (AMCA), 7-diethylamino-3-[(4'-(iodoacetyl)amino)phenyl]-4-methylcoumarin (DCIA), Alexa fluor dyes (Molecular Probes), and the like; 4,4-difluoro-4-bora-3a,4a-diaza-s-indacene (BODIPY™) and derivatives thereof (Molecular Probes; Eugene Oreg.); pyrenes and sulfonated pyrenes such as Cascade Blue™ and derivatives thereof, including 8-methoxypyrene-1,3,6-trisulfonic acid, and the like; pyridyloxazole derivatives and dapoxyl derivatives (Molecular Probes); Lucifer Yellow (3,6-disulfonate-4-amino-naphthalimide) and derivatives thereof; CyDye™ fluorescent dyes (Amersham/GE Healthcare Life Sciences; Piscataway NJ), and the like. Exemplary chromophores include, but are not limited to, phenolphthalein, malachite green, nitroaromatics such as nitrophenyl, diazo dyes, dabsyl (4-dimethylaminoazobenzene-4'-sulfonyl), and the like.

Well known methods such as microscopy or spectroscopy can be utilized to visualize chromogenic or fluoroscent detectable signals associated with the respective target nucleic acids. In general, either chromogenic substrates or fluorogenic substrates, or chromogenic or fluorescent labels, will be utilized for a particular assay, if different labels are used in the same assay, so that a single type of instrument can be used for detection of nucleic acid targets in the same sample.

In one embodiment, label probes are directly hybridized to the target nucleic acid molecules. As discussed above, in such an embodiment the label probe itself comprises a nucleic acid target binding region, such as a complementary oligonucleotide, and binds directly to the target nucleic acid. Alternatively, the label probe can be bound to the target nucleic acid indirectly using an intermediary complex (see also WO 2013/134442 and WO 2013/152295). In one such embodiment, the intermediary complex can be a single molecule such as a single nucleic acid molecule. For example, an intermediary complex that is a single molecule can comprise a region that is capable of specifically and selectively attaching or binding to a target nucleic acid. This is similar to a label probe that is designed to attach or bind to a target nucleic acid, except that the binding to the nucleic acid target is mediated by the intermediary molecule. The intermediary molecule also can comprise a region that is capable of specifically and selectively binding to a label probe. In such a case, the label probe and intermediary molecule are designed to have binding regions such as oligonucleotide regions that are complementary to each other. Such a configuration thereby associates the enzyme label to a target nucleic acid specifically and selectively through an intermediary molecule. A second target nucleic acid can be specifically and selectively associated with the same label or a distinct label through a second intermediary complex. The second intermediary complex comprises a first region that can specifically and selectively bind to the target nucleic acid and a second region that can specifically and selectively bind to the same label probe or a second label probe. Thus, the label probes can be bound to the target nucleic acid molecules by a first and second intermediary complex. Further, the intermediary complex can comprise a molecule comprising a first region complementary to the target nucleic acid and the same or a different molecule comprising at least one second region complementary to the label probe. Further still, the intermediary complex can comprise an assembly of multiple molecules, of which one or more components each comprising one or more regions complementary to the target nucleic acid and one or more other components each comprising one or more regions complementary to the label probe. In this way, the intermediary complex can be an amplifying structure allowing multiple label probes to be attached to a region on a target nucleic acid. In general, the intermediary complex comprises one or more nucleic acid molecules to take advantage of the specific and selective binding capabilities of nucleic acid interactions.

As described herein, a sample can be contacted with label probes, either the same or distinct, as disclosed herein. The label probes are bound to the target nucleic acid molecules by hybridization of complementary regions on the target nucleic acid and either the label probe or the intermediary complex, as discussed herein.

As described herein, the label probe used in methods of the invention can comprise a target nucleic acid binding region comprising an oligonucleotide, which is complementary to the target nucleic acid or complementary to a component of an intermediary complex that can bind to the target nucleic acid. In such a configuration of a label probe, the oligonucleotide portion is coupled to an enzyme or non-enzyme label. The coupling of the enzyme or non-enzyme label to the oligonucleotide in the label probe can be covalent, using well known chemical coupling methods (see, for example, Hermanson, Bioconjugate Techniques, Academic Press, San Diego (1996)). Alternatively, a high affinity interaction such as biotin/avidin, where biotin or avidin is coupled to the oligonucleotide and enzyme or non-enzyme label in the label probe, can be used to couple the enzyme or non-enzyme label to the oligonucleotide in the label probe (see US publication 2012/0100540). It is understood that the coupling of the enzyme or non-enzyme label to the oligonucleotide in the label probe is of sufficient affinity, such as through an interaction such as biotin/avidin, or is covalent such that the enzyme or non-enzyme label remains attached to the oligonucleotide throughout various reactions and assay conditions of the methods of the invention.

In still another embodiment, the methods of the invention can employ an intermediary complex, where the intermediary complex comprises multiple molecules rather than a single molecule. Such an intermediary complex is particularly useful for amplifying the detectable signal, providing higher sensitivity detection of target nucleic acids. Such methods for amplifying a signal are described, for example, in U.S. Patent Nos. 5,635,352, 5,124,246, 5,710,264, 5,849,481, and 7,709,198, and U.S. publications 2008/0038725 and 2009/0081688, as well as WO 2007/001986 and WO 2012/054795.

Thus, in a particular embodiment the intermediary complex can comprise a target probe, amplifier and preamplifier. In addition, a plurality of label probes can be hybridized to one or more amplifiers, a plurality of amplifiers can be hybridized to a preamplifier, and the preamplifier can be hybridized to a target probe.

In yet another embodiment, a configuration can be used, where the intermediary complex comprises a target probe, preamplifier and amplifier. Rather than using a single target probe to bind the preamplifier to the target nucleic acid, two or more target probes are used to bind the preamplifier to the target nucleic acid. In general, the advantages of such a configuration can be realized using two target probes, that is, a pair of target probes to bind a preamplifier to the target nucleic acid. Such a configuration and its advantages for increasing sensitivity and decreasing background are described, for example, in U.S. Patent No. 7,709,198, U.S. publications 2008/0038725 and 2009/0081688, and WO 2007/001986, WO 2007/002006, WO 2013/134442 and WO 2013/152295. U.S. Patent No. 7,709,198 and U.S. publications 2008/0038725 and 2009/0081688 additionally describe details for selecting characteristics of the target probes, including target probe pairs, including length, orientation, hybridization conditions, and the like, as discussed below in more detail.

It is understood by those skilled in the art that any of a number of suitable samples can be used for detecting target nucleic acids using methods of the invention. The sample for use in methods of the invention will generally be a biological sample or tissue sample. Such a sample can be obtained from a biological subject, including a sample of biological tissue or fluid origin that is collected from an individual or some other source of biological material such as a biopsy. A biological sample also includes samples from a region of a biological subject containing or suspected of containing a cervical lesion, precancerous or cancer cells or tissues, for example, a tissue biopsy, cervical cell smear (Pap smear). Such samples can be, but are not limited to, tissues, tissue fractions and/or cells isolated from an organism such as a mammal, in particular a human. In a particular embodiment, the tissue section is formalin fixed, paraffin embedded (FFPE).

The sample or biological specimen can be processed by any of a number of routine methods used for handling biological samples. For example, the cells can be used as isolated or processed, for example, as tissue slices or other tissue specimens, including fixing cells such as with formalin fixed and paraffin embedded (FFPE) tissue, fresh frozen tissue, white blood cells, and the like. Methods for processing cervical samples are well known in the art (see Sellors and Sankaranarayanan, *supra,* 2003). Methods for processing tissue or cell samples for *in* situ hybridization are well known to those skilled in the art (see, for example, Stoler, Clinics in Laboratory Medicine 10(1):215-236 (1990); In situ hybridization. A practical approach, Wilkinson, ed., IRL Press, Oxford (1992); Schwarzacher and Heslop-Harrison, Practical in situ hybridization, BIOS Scientific Publishers Ltd, Oxford (2000);). Methods for processing samples for analysis of cervical tissue, including tissue biopsy and cytology samples, are well known in the art (see, for example, Cecil Textbook of Medicine, Bennett and Plum, eds., 20th ed., WB Saunders, Philadelphia (1996); Colposcopy and Treatment of Cervical Intraepithelial Neoplasia: A Beginner's Manual, Sellors and Sankaranarayanan, eds., International Agency for Research on Cancer, Lyon, France (2003); Kalaf and Cooper, J. Clin. Pathol. 60:449-455 (2007); Brown and Trimble, Best Pract. Res. Clin. Obstet. Gynaecol. 26:233-242 (2012); Waxman et al., Obstet. Gynecol. 120:1465-1471 (2012); Cervical Cytology Practice Guidelines TOC, Approved by the American Society of Cytopathology (ASC) Executive Board, November 10, 2000)).

As disclosed herein, the methods of the invention provide for a convenient assay method to detect multiple target nucleic acids, in particular high risk HPV subtypes, in the same sample. In addition to direct coupling of a label probe with an enzyme or non-enzyme label, in another embodiment an antibody can be used as an intermediary to label the target nucleic acid. Methods have been previously described for detecting two DNA targets simultaneously, such as a two-color chromogenic ISH (CISH) assay (US2011/033176), in which probes against two different genes are conjugated with a hapten such as digoxigenin (DIG) or dinitrophenyl (DNP). Then, anti-DIG and anti-DNP antibodies, or other suitable anti-hapten antibodies, that are conjugated with different enzymes, such as horseradish peroxidase and alkaline phosphatase, are used to generate different color precipitates. While the assay allows visualization of the two targets, the visualization occurs using an antibody to direct the enzyme to the target nucleic acid. Such antibody based ISH assays can be similarly applied to the methods of the invention, as disclosed herein.

As disclosed herein, by utilizing a label probe comprising nucleic acid to bind to the target nucleic acid, a high level of specificity and selectivity can be achieved by using appropriate *in silico* oligonucleotide design and by selecting and controlling hybridization conditions using well known methods. Such a system can be optimized for selectivity and specificity and minimized for non-specific binding between nucleic acid targets.

The disclosure additionally provides kits comprising components for performing methods of the invention. In one embodiment, the disclosure provides a kit for detecting high risk HPV subtypes, as disclosed herein, including antisense and sense probes to the HPV subtypes. The kit can contain agents for conducting an RNA *in situ* hybridization assay including at least one probe set which is designed to hybridize to one or more HPV subtypes; and a signal amplification system. Generally such a kit would be provided in a suitable container. Optionally the kit can further comprise at least one negative control probe set. In a particular embodiment, the kit contains reagents for performing and *in situ* hybridization assay as an RNAscope® assay, as disclosed herein and described previously (U.S. Patent No. 7,709,198, U.S. publications 2008/0038725 and 2009/0081688, and WO 2007/001986 and WO 2007/002006).

Such kits, in addition to the components described above, can optionally include, for example, label probes, amplifiers, preamplifiers, target probes, and the like. Such components can be designed as for any of the configurations disclosed herein. A kit of the invention can, for example, comprise label probes comprising the same label or distinct labels and, if the label includes and enzyme, suitable chromogenic or fluorogenic substrates for the enzyme, for carrying out methods of the invention. The kits can contain non-target-specific components, which can be used for detection of any desired target nucleic acid, with a target specific binding component to be designed separately by the user of the kit, for example probes to high risk HPV subtypes, as disclosed herein. Alternatively, the kit can be designed to contain components that include binding agents for one or more particular target nucleic acids such high risk HPV subtypes. The kit can additionally include instructions for using the components of the kit to carry out methods of the invention.

As described herein, embodiments of the disclosure can include the use of target probes. Such a configuration and its advantages for increasing sensitivity and decreasing background are described, for example, in U.S. Patent No. 7,709,198, U.S. publications 2008/0038725 and 2009/0081688, and WO 2007/001986 and WO 2007/002006. U.S. Patent No. 7,709,198 and U.S. publications 2008/0038725 and 2009/0081688 additionally describe details for selecting characteristics of the target probes, including target probe pairs, including length, orientation, hybridization conditions, and the like. One skilled in the art can readily identify suitable configurations based on the teachings 7,709,198, U.S. publications 2008/0038725 and 2009/0081688, and WO 2007/001986 and WO 2007/002006. In the description provided below, based on the disclosure herein and the teachings in 7,709,198, U.S. publications 2008/0038725 and 2009/0081688, and WO 2007/001986 and WO 2007/002006, one skilled in the art will understand that the term "label extender," as used in these references and as discussed below, can be used interchangeably with the term "target probe," as described herein.

Briefly, a first general class of embodiments includes methods of detecting two or more nucleic acids of interest. In the methods, a sample comprising or suspected of comprising the nucleic acids of interest, two or more subsets of m label extenders (target probes), wherein m is at least two, and a label probe system are provided. Each subset of m label extenders is capable of hybridizing to one of the nucleic acids of interest. For example, the nucleic acid of interest can be one or more high risk HPV subtypes. The label probe system comprises a label, and a component of the label probe system is capable of hybridizing simultaneously to at least two of the m label extenders in a subset. Each nucleic acid of interest is hybridized to its corresponding subset of m label extenders, and the label probe system is hybridized to the m label extenders. The presence or absence of the label is then detected. Since the label is associated with the nucleic acid(s) of interest via hybridization of the label extenders and label probe system, the presence or absence of the label is correlated with the presence or absence of the nucleic acid(s) of interest and thus in the original sample. The label probe system can optionally include a preamplifier, an amplification multimer, and a label probe, wherein the preamplifier is capable of hybridizing simultaneously to the at least two of the m label extenders and to a plurality of amplification multimers, and wherein the amplification multimer is capable of hybridizing simultaneously to the preamplifier and to a plurality of label probes.

As noted above, a component of the label probe system is capable of hybridizing simultaneously to at least two of the m label extenders in a subset. Typically, the component of the label probe system that hybridizes to the two or more label extenders is an amplification multimer or preamplifier. Thus, in one aspect, the label probe system comprises an amplification multimer or preamplifier, which amplification multimer or preamplifier is capable of hybridizing to the at least two label extenders, and the label probe system is hybridized to the m label extenders at a hybridization temperature, which hybridization temperature is greater than a melting temperature Tₘ of a complex between each individual label extender and the amplification multimer or preamplifier. The hybridization temperature is typically about 5° C or more greater than the Tm, for example, about 7° C or more, about 10° C or more, about 12° C or more, about 15° C or more, about 17° C or more, or even about 20° C or more greater than the Tm.

Each label extender typically comprises a polynucleotide sequence L-1 that is complementary to a polynucleotide sequence in the corresponding nucleic acid of interest (also referred to herein as a T section complementary to a target nucleic acid) and a polynucleotide sequence L-2 that is complementary to a polynucleotide sequence in the component of the label probe system (also referred to herein as an L section complementary to a label probe system). In one class of embodiments, the m label extenders in a subset each have L-1 5' of L-2 or each have L-1 3' of L-2. The length of L-2 can vary. For example, sequence L-2 can be 20 nucleotides or less in length, for example, L-2 can be between 9 and 17 nucleotides in length or between 12 and 15 or between 13 and 15 nucleotides in length.

Another general class of embodiments provides methods of detecting one or more nucleic acids using a label extender configuration. In the methods, a sample comprising or suspected of comprising the nucleic acids of interest, one or more subsets of m label extenders, wherein m is at least two, and a label probe system are provided. Each subset of m label extenders is capable of hybridizing to one of the nucleic acids of interest. The label probe system comprises a label, and a component of the label probe system (for example, a preamplifier or an amplification multimer) is capable of hybridizing simultaneously to at least two of the m label extenders in a subset. Each label extender comprises a polynucleotide sequence L-1 that is complementary to a polynucleotide sequence in the corresponding nucleic acid of interest and a polynucleotide sequence L-2 that is complementary to a polynucleotide sequence in the component of the label probe system, and the at least two label extenders (for example, the m label extenders in a subset) each have L-1 5' of L-2 or each have L-1 3' of L-2.

Each nucleic acid of interest is hybridized to its corresponding subset of m label extenders, and the label probe system is hybridized to the m label extenders at a hybridization temperature. The hybridization temperature can be greater than a melting temperature Tm of a complex between each individual label extender and the component of the label probe system. Since the label is associated with the nucleic acid(s) of interest via hybridization of the label extenders and label probe system, the presence or absence of the nucleic acid(s) of interest can be determined.

Yet another general class of embodiments provides methods of capturing a label to a first nucleic acid of interest in a multiplex assay in which two or more nucleic acids of interest are to be detected. In the methods, a sample comprising the first nucleic acid of interest and also comprising or suspected of comprising one or more other nucleic acids of interest is provided. A first subset of m label extenders, wherein m is at least two, and a label probe system comprising the label are also provided. The first subset of m label extenders is capable of hybridizing to the first nucleic acid of interest, and a component of the label probe system is capable of hybridizing simultaneously to at least two of the m label extenders in the first subset. The first nucleic acid of interest is hybridized to the first subset of m label extenders, and the label probe system is hybridized to the m label extenders, thereby capturing the label to the first nucleic acid of interest.

Yet another general class of embodiments provides methods of capturing a label to a nucleic acid of interest. In the methods, m label extenders, wherein m is at least two, are provided. The m label extenders are capable of hybridizing to the nucleic acid of interest. A label probe system comprising the label is also provided. A component of the label probe system is capable of hybridizing simultaneously to at least two of the m label extenders. Each label extender comprises a polynucleotide sequence L-1 that is complementary to a polynucleotide sequence in the nucleic acid of interest and a polynucleotide sequence L-2 that is complementary to a polynucleotide sequence in the component of the label probe system, and the m label extenders each have L-1 5' of L-2 or wherein the m label extenders each have L-1 3' of L-2. The nucleic acid of interest is hybridized to the m label extenders, and the label probe system is hybridized to the m label extenders at a hybridization temperature, thereby capturing the label to the nucleic acid of interest. Preferably, the hybridization temperature is greater than a melting temperature Tm of a complex between each individual label extender and the component of the label probe system.

A "label extender" or "LE" is a polynucleotide that is capable of hybridizing to a nucleic acid of interest and to a label probe system. Such a label extender is also referred to herein as a target probe. The label extender typically has a first polynucleotide sequence L-1, which is complementary to a polynucleotide sequence of the nucleic acid of interest, and a second polynucleotide sequence L-2, which is complementary to a polynucleotide sequence of the label probe system (for example, L-2 can be complementary to a polynucleotide sequence of an amplification multimer, a preamplifier, a label probe, or the like). The label extender is generally single-stranded.

A "label probe system" comprises one or more polynucleotides that collectively comprise a label and at least two polynucleotide sequences M-1, each of which is capable of hybridizing to a label extender. The label provides a signal, directly or indirectly. Polynucleotide sequence M-1 is typically complementary to sequence L-2 in the label extenders. The at least two polynucleotide sequences M-1 are optionally identical sequences or different sequences. The label probe system can include a plurality of label probes (for example, a plurality of identical label probes) and an amplification multimer; it optionally also includes a preamplifier or the like, or optionally includes only label probes, for example.

An "amplification multimer" is a polynucleotide comprising a plurality of polynucleotide sequences M-2, typically (but not necessarily) identical polynucleotide sequences M-2. Polynucleotide sequence M-2 is complementary to a polynucleotide sequence in the label probe. The amplification multimer also includes at least one polynucleotide sequence that is capable of hybridizing to a label extender or to a nucleic acid that hybridizes to the label extender, for example, a preamplifier. For example, the amplification multimer optionally includes at least one (and generally at least two) polynucleotide sequence(s) M-1, optionally identical sequences M-1; polynucleotide sequence M-1 is typically complementary to polynucleotide sequence L-2 of the label extenders. Similarly, the amplification multimer optionally includes at least one polynucleotide sequence that is complementary to a polynucleotide sequence in a preamplifier. The amplification multimer can be, for example, a linear or a branched nucleic acid. As noted for all polynucleotides, the amplification multimer can include modified nucleotides and/or nonstandard internucleotide linkages as well as standard deoxyribonucleotides, ribonucleotides, and/or phosphodiester bonds. Suitable amplification multimers are described, for example, in U.S. Pat. No. 5,635,352, U.S. Pat. No. 5,124,246, U.S. Pat. No. 5,710,264, U.S. Pat. No. 5,849,481 and U.S. Pat. No. 7,709,198.

Signal amplification begins with the binding of the LEs to the target mRNA or DNA. An amplification multimer is then typically hybridized to the LEs. The amplification multimer generally has multiple copies of a sequence that is complementary to a label probe and can be a branched-chain nucleic acid; for example, the amplification multimer can be a branched, forked, or comb-like nucleic acid or a linear nucleic acid. A label, for example, an enzyme or non-enzyme label, is covalently attached to each label probe.

In the preceding embodiment, the amplification multimer and the label probes comprise a label probe system. In another embodiment, the label probe system also comprises a preamplifier, for example, as described in U.S. Pat. No. 5,635,352, U.S. Pat. No. 5,681,697 and U.S. Pat. No. 7,709,198, which further amplifies the signal from a single target mRNA. In yet another example, the label extenders hybridize directly to the label probes and no amplification multimer or preamplifier is used, so the signal from a single target mRNA or DNA molecule is only amplified by the number of distinct label extenders that hybridize to that mRNA or DNA.

Among other aspects, the present invention provides multiplex bDNA assays that can be used for simultaneous detection of two or more target nucleic acids. Similarly, one aspect of the present invention provides bDNA assays, singleplex or multiplex, that have reduced background from nonspecific hybridization events.

In general, in the assays of the invention, two or more label extenders are used to capture a single component of the label probe system (for example, a preamplifier or amplification multimer). The assay temperature and the stability of the complex between a single LE and the component of the label probe system (for example, the preamplifier or amplification multimer) can be controlled such that binding of a single LE to the component is not sufficient to stably associate the component with a nucleic acid to which the LE is bound, whereas simultaneous binding of two or more LEs to the component can capture it to the nucleic acid. Requiring such cooperative hybridization of multiple LEs for association of the label probe system with the nucleic acid(s) of interest results in high specificity and low background from cross-hybridization of the LEs with other, non-target nucleic acids.

For an assay to achieve high specificity and sensitivity, it preferably has a low background, resulting, for example, from minimal cross-hybridization. Such low background and minimal cross-hybridization are typically substantially more difficult to achieve in a multiplex assay than a single-plex assay, because the number of potential nonspecific interactions are greatly increased in a multiplex assay due to the increased number of probes used in the assay (for example, the greater number of CEs and LEs). Requiring multiple simultaneous LE-label probe system component interactions for the capture of the label probe system to a target nucleic acid minimizes the chance that nonspecific capture will occur, even when some nonspecific CE-LE or LE-CP interactions, for example, do occur. This reduction in background through minimization of undesirable cross-hybridization events thus facilitates multiplex detection of the nucleic acids of interest.

The label probe system optionally includes an amplification multimer and a plurality of label probes, wherein the amplification multimer is capable of hybridizing to the label extenders and to a plurality of label probes. In another aspect, the label probe system includes a preamplifier, a plurality of amplification multimers, and a plurality of label probes, wherein the preamplifier hybridizes to the label extenders, and the amplification multimers hybridize to the preamplifier and to the plurality of label probes. As another example, the label probe system can include only label probes, which hybridize directly to the label extenders. In one class of embodiments, the label probe comprises the label, for example, a covalently attached label. In other embodiments, the label probe is configured to bind a label; for example, a biotinylated label probe can bind to a streptavidin-associated label, as disclosed herein.

Each label extender typically includes a polynucleotide sequence L-1 that is complementary to a polynucleotide sequence in the corresponding nucleic acid of interest and a polynucleotide sequence L-2 that is complementary to a polynucleotide sequence in the component of the label probe system (for example, the preamplifier or amplification multimer). It will be evident that the amount of overlap between each individual label extender and the component of the label probe system (i.e., the length of L-2 and M-1) affects the Tm of the complex between the label extender and the component, as does, for example, the GC base content of sequences L-2 and M-1. Optionally, all the label extenders have the same length sequence L-2 and/or identical polynucleotide sequences L-2. Alternatively, different label extenders can have different length and/or sequence polynucleotide sequences L-2. It will also be evident that the number of label extenders required for stable capture of the component to the nucleic acid of interest depends, in part, on the amount of overlap between the label extenders and the component (i.e., the length of L-2 and M-1) and can be readily determined by one skilled in the art.

Stable capture of the component of the label probe system by the at least two label extenders, for example, while minimizing capture of extraneous nucleic acids, can be achieved, for example, by balancing the number of label extenders that bind to the component, the amount of overlap between the label extenders and the component (the length of L-2 and M-1), and/or the stringency of the conditions under which the label extenders and the component are hybridized. Appropriate combinations of the amount of complementarity between the label extenders and the component of the label probe system, number of label extenders binding to the component, and stringency of hybridization can, for example, be determined experimentally by one of skill in the art. For example, a particular number of label extenders and a particular set of hybridization conditions can be selected, while the number of nucleotides of complementarity between the label extenders and the component is varied until hybridization of the label extenders to a nucleic acid captures the component to the nucleic acid while hybridization of a single label extender does not efficiently capture the component. Stringency can be controlled, for example, by controlling the formamide concentration, chaotropic salt concentration, salt concentration, pH, organic solvent content, and/or hybridization temperature.

As noted, the Tm of any nucleic acid duplex can be directly measured, using techniques well known in the art. For example, a thermal denaturation curve can be obtained for the duplex, the midpoint of which corresponds to the Tm. It will be evident that such denaturation curves can be obtained under conditions having essentially any relevant pH, salt concentration, solvent content, and/or the like.

Typically, the component of the label probe system (for example, the amplification multimer or preamplifier) is capable of hybridizing simultaneously to two of the m label extenders in a subset, although it optionally hybridizes to three, four, or more of the label extenders. In one class of embodiments, for example, embodiments in which two (or more) label extenders bind to the component of the label probe system, sequence L-2 is 20 nucleotides or less in length. For example, L-2 can be between 9 and 17 nucleotides in length, for example, between 12 and 15 nucleotides in length, between 13 and 15 nucleotides in length, or between 13 and 14 nucleotides in length. As noted, m is at least two, and can be at least three, at least five, at least 10, or more. m can be the same or different from subset to subset of label extenders.

The label extenders can be configured in any of a variety ways. For example, the two label extenders that hybridize to the component of the label probe system can assume a cruciform arrangement, with one label extender having L-1 5' of L-2 and the other label extender having L-1 3' of L-2. A configuration in which either the 5' or the 3' end of both label extenders hybridizes to the nucleic acid while the other end binds to the component yields stronger binding of the component to the nucleic acid than does a cruciform arrangement of the label extenders. Thus, in one class of embodiments, the at least two label extenders (for example, the m label extenders in a subset) each have L-1 5' of L-2 or each have L-1 3' of L-2. For example, L-1, which hybridizes to the nucleic acid of interest, can be at the 5' end of each label extender, while L-2, which hybridizes to the component of the label probe system, is at the 3' end of each label extender (or vice versa). L-1 and L-2 are optionally separated by additional sequence. In one exemplary embodiment, L-1 is located at the 5' end of the label extender and is about 20-30 nucleotides in length, L-2 is located at the 3' end of the label extender and is about 13-14 nucleotides in length, and L-1 and L-2 are separated by a spacer (for example, 5 Ts).

The methods of the invention as disclosed herein can also be performed with the additional embodiments described below. The steps of such a method can include, for example, providing a sample comprising the cell, which cell comprises or is suspected of comprising one or more nucleic acid targets, for example, a sample comprising cervical tissue or cervical cells; providing for each nucleic acid target a label probe system comprising label; providing for each nucleic acid target a label extender system comprising two or more different label extenders, wherein each of the two or more label extenders comprises a section T complementary to a section on the nucleic acid target and a section L complementary to a section on a component of the label probe system, and wherein the T sections are complementary to non-overlapping regions of the nucleic acid target, and the L sections are complementary to non-overlapping regions of the component of the label probe system; hybridizing, in the cell, the label extenders system to the nucleic acid target, when present in the cell, thereby providing hybridization of two or more different label extenders to a single copy of the nucleic acid target molecule; capturing, in the cell and in the presence of cellular non-target nucleic acids, the label probe system to the label extender system hybridized to the nucleic acid target molecule, thereby capturing the label to the nucleic acid target, wherein the capturing occurs by simultaneously hybridizing the at least two different label extenders to a single molecule of the component of the label probe system that is complementary to the label extenders; and detecting a signal from the label.

An embodiment can include hybridizing the at least two different label extenders to the single molecule of the component of the label probe system that is complementary to the label extenders at a hybridization temperature that is greater than a melting temperature Tm of a complex between each individual label extender and the component of the label probe system, for example, the label probe, amplifier or preamplifier. In another embodiment, the at least two different label extenders hybridize to adjacent sections on the nucleic acid target. Also, hybridizing the two or more label extenders to the single molecule of the nucleic acid target can be performed at a hybridization temperature that is greater than a melting temperature Tm of a complex between each individual label extender and the nucleic acid target. Another embodiment can further comprise providing one or more blocking probes capable of hybridizing to regions of the nucleic acid target not occupied by the label extenders. In still another embodiment, each hybridizing or capturing step is accomplished for all the nucleic acid targets at the same time. In a further embodiment, the one or more nucleic acid targets can comprise a reference nucleic acid, and wherein the method comprises normalizing the signal of the one or more nucleic acid targets to the signal of the reference nucleic acid. Still a further embodiment can comprise the step of correlating the intensity of the signal of each nucleic acid target with a quantity of the corresponding nucleic acid target present in the cell.

In a particular embodiment, each label extender is in a "Z" configuration, wherein the Z configuration comprises the T section (that binds to the target nucleic acid) of the label extender at the 5' end and the L section (that binds to the label probe system) of the label extender at the 3' end, or the T section of the label extender at the 3' end and the L section of the label extender at the 5' end. In another embodiment, the label probe system comprises (A) (i) a label probe comprising one or more labels, or (B) (i) one or more label probes and (ii) an amplifier capable of hybridizing to one or more of the label probes, or (C) (i) one or more label probes, (ii) one or more amplifiers, and (iii) a preamplifier capable of hybridizing to one or more of the amplifiers. In a further embodiment, the label is captured to the nucleic acid target molecule by hybridization of the label probe to the at least two different label extenders hybridized to the nucleic acid target molecule. In still a further embodiment, the label is captured to the nucleic acid target molecule by hybridization of the one or more label probes to the amplifier molecule hybridized to the at least two different label extenders hybridized to the nucleic acid target molecule. In yet another embodiment, the label is captured to the nucleic acid target molecule by hybridization of the one or more label probes to the one or more amplifiers hybridized to the preamplifier molecule hybridized to the at least two different label extenders hybridized to the nucleic acid target molecule. In a still further embodiment, a plurality of label probe systems are provided for each target nucleic acid and wherein two or more label probe systems are captured to the target nucleic acid by using the label extender system comprising two or more different label extenders and wherein each label extender in the label extender system comprises a T section that is complementary to non-overlapping regions of the nucleic acid target. In yet another embodiment, three or more label probe systems are captured to the target nucleic acid by the label extender system.

The methods of the disclosure can additionally include further aspects as described below. For example, the methods can further include a method of detecting one or more nucleic acids of interest, the method comprising: a) providing a sample comprising or suspected of comprising the one or more nucleic acids of interest; b) labeling those nucleic acids of interest present in the sample using the methods disclosed herein; c) providing one or more subsets of m label extenders, wherein m is at least two, wherein each subset of m label extenders is capable of hybridizing to one of the nucleic acids of interest; d) providing a label probe system comprising a label, wherein a component of the label probe system is capable of hybridizing simultaneously to at least two of the m label extenders in a subset, wherein each of the at least two label extenders comprises a polynucleotide sequence L-1 that is complementary to a polynucleotide sequence in the corresponding nucleic acid of interest and comprises a polynucleotide sequence L-2 that is complementary to a polynucleotide sequence in the component of the label probe system, wherein the at least two label extenders all have L-1 5' of L-2 or all have L-1 3' of L-2, and wherein the m label extenders in the subset are complementary to nonoverlapping sequences in the corresponding nucleic acid of interest; e) hybridizing any nucleic acid of interest captured to its corresponding subset of m label extenders; f) hybridizing the label probe system to the m label extenders at a hybridization temperature, which hybridization temperature is greater than a melting temperature Tm of a complex between each individual label extender and the component of the label probe system; and g) detecting the presence or absence of the label on the target nucleic acid.

In a particular embodiment, the label probe system comprises a preamplifier, an amplification multimer and a label probe; wherein the preamplifier is capable of hybridizing simultaneously to the at least two of the m label extenders and to a plurality of amplification multimers: wherein the amplification multimer is capable of hybridizing simultaneously to the preamplifier and to a plurality of label probes; and wherein the label probe comprises the label. In another embodiment, one or more nucleic acids of interest comprise two or more different nucleic acids of interest, which different nucleic acids of interest are different nucleic acid molecules, and wherein the one or more subsets of m label extenders comprise two or more different subsets of m label extenders. In a particular embodiment, the label probe system comprises an amplification multimer or preamplifier, which amplification multimer or preamplifier is capable of hybridizing to the at least two label extenders.

In an embodiment, the hybridization temperature is about 5° C or more greater than the Tm of a complex between each individual label extender and the component of the label probe system. Alternatively, the hybridization temperature is about 7° C or more, about 10° C or more, about 12° C or more, about 15° C or more, about 17° C or more, or about 20° C or more greater than the Tm of a complex between each individual label extender and the component of the label probe system. In another embodiment, the polynucleotide sequence L-2 is 20 nucleotides or less in length. Alternatively, L-2 is between 9 and 17 nucleotides in length. Alternatively, L-2 is between 13 and 15 nucleotides in length.

In another embodiment, each of the at least two label extenders has L-1 at its 5' end and L-2 at its 3' end. In still another embodiment, each of the at least two label extenders has L-1 at its 3' end and L-2 at its 5' end. In still another embodiment, the m label extenders in a subset all have L-1 5' of L-2 or all have L-1 3' of L-2. In another embodiment, the label probe system comprises an amplification multimer and a label probe; wherein the amplification multimer is capable of hybridizing simultaneously to the at least two of the m label extenders and to a plurality of label probes; and wherein the label probe comprises the label. In another embodiment, the label probe system comprises a label probe; wherein the label probe is capable of hybridizing simultaneously to the at least two of the m label extenders; and wherein the label probe comprises the label. In another embodiment, the component of the label probe system is capable of hybridizing simultaneously to two of the m label extenders in a subset.

A method of the disclosure can further involve capturing a label to a nucleic acid of interest, by a) providing m label extenders, wherein in is at least two, wherein the m label extenders are capable of hybridizing to nonoverlapping sequences in the nucleic acid of interest; b) providing a label probe system comprising the label, wherein a component of the label probe system is capable of hybridizing simultaneously to at least two of the m label extenders, wherein each of the at least two label extenders comprises a polynucleotide sequence L-1 that is complementary to a polynucleotide sequence in the nucleic acid of interest and comprises a polynucleotide sequence L-2 that is complementary to a polynucleotide sequence in the component of the label probe system, and wherein the at least two label extenders all have L-1 5' of L-2 or all have L-1 3' of L-2; c) hybridizing the nucleic acid of interest to the m label extenders; and d) hybridizing the label probe system to the in label extenders at a hybridization temperature, which hybridization temperature is greater than a melting temperature Tm of a complex between each individual label extender and the component of the label probe system, thereby capturing the label to the nucleic acid of interest.

In an embodiment, the m label extenders all have L-1 5' of L-2 or all have L-1 3' of L-2. In an embodiment, the label probe system comprises an amplification multimer or preamplifier, which amplification multimer or preamplifier is capable of hybridizing to at least two of the m label extenders. In an embodiment, the label probe system comprises a preamplifier, an amplification multimer and a label probe; wherein the preamplifier is capable of hybridizing simultaneously to at least two of the m label extenders and to a plurality of amplification multimers; wherein the amplification multimer is capable of hybridizing simultaneously to the preamplifier and to a plurality of label probes; and wherein the label probe comprises the label. In an embodiment, the label probe system comprises an amplification multimer and a label probe; wherein the amplification multimer is capable of hybridizing simultaneously to the at least two of the m label extenders and to a plurality of label probes; and wherein the label probe comprises the label. In an embodiment, the label probe system comprises a label probe; wherein the label probe is capable of hybridizing simultaneously to the at least two of the m label extenders; and wherein the label probe comprises the label. In an embodiment, the component of the label probe system is capable of hybridizing simultaneously to two of the m label extenders. In an embodiment, the hybridization temperature is about 5° C or more greater than the Tm of a complex between each individual label extender and the component of the label probe system. In an embodiment, the hybridization temperature is about 7° C or more, about 10° C or more, about 12° C or more, about 15° C or more, about 17° C or more, or about 20° C or more greater than the Tm of a complex between each individual label extender and the component of the label probe system. In an embodiment, the polynucleotide sequence L-2 is 20 nucleotides or less in length. In an embodiment, L-2 is between 9 and 17 nucleotides in length. In an embodiment, L-2 is between 13 and 15 nucleotides in length. In an embodiment, each of the at least two label extenders has L-1 at its 5' end and L-2 at its 3' end. In an embodiment, each of the at least two label extenders has L-1 at its 3' end and L-2 at its 5' end.

A further embodiment can include a method of detecting one or more nucleic acids of interest, by a) providing a sample comprising the one or more nucleic acids of interest; b) labeling the nucleic acids of interest using the methods disclosed herein; c) providing a label probe system comprising a label; d) for each of the nucleic acids of interest, providing a subset of m label extenders, wherein m is at least two, wherein the subset of m label extenders is capable of hybridizing to that nucleic acid of interest, wherein a component of the label probe system is capable of hybridizing simultaneously to at least two of the m label extenders in the subset, wherein each of the at least two label extenders comprises a polynucleotide sequence L-1 that is complementary to a polynucleotide sequence in the nucleic acid of interest and comprises a polynucleotide sequence L-2 that is complementary to a polynucleotide sequence in the component of the label probe system, and wherein the at least two label extenders all have L-1 5' of L-2 or all have L-1 3' of L-2; e) hybridizing each nucleic acid of interest to its corresponding subset of m label extenders; f) hybridizing the label probe system to the label extenders at a hybridization temperature, which hybridization temperature is greater than a melting temperature Tm of a complex between each individual label extender and the component of the label probe system, whereby, for each of the nucleic acids of interest, a single copy of each of the at least two label extenders hybridizes simultaneously to a single copy of the nucleic acid of interest and to a single copy of the component of the label probe system; and g) detecting the presence or absence of the label on the target nucleic acid.

In yet another embodiment, a method can include capturing a label to a nucleic acid of interest, by: a) providing m label extenders, wherein m is at least two, wherein the m label extenders are capable of hybridizing to the nucleic acid of interest; b) providing a label probe system comprising the label, wherein a component of the label probe system is capable of hybridizing simultaneously to at least two of the m label extenders, wherein each of the at least two label extenders comprises a polynucleotide sequence L-1 that is complementary to a polynucleotide sequence in the nucleic acid of interest and comprises a polynucleotide sequence L-2 that is complementary to a polynucleotide sequence in the component of the label probe system, and wherein the at least two label extenders all have L-1 5' of L-2 or all have L-1 3' of L-2; c) hybridizing the nucleic acid of interest to the m label extenders; and d) hybridizing the label probe system to the m label extenders at a hybridization temperature, which hybridization temperature is greater than a melting temperature Tm of a complex between each individual label extender and the component of the label probe system, whereby a single copy of each of the at least two label extenders hybridizes simultaneously to a single copy of the nucleic acid of interest and to a single copy of the component of the label probe system, thereby capturing the label to the nucleic acid of interest.

The following examples are intended to illustrate but not limit the present invention.

### EXAMPLE I

### HPV Detection in Caski Cells

This example describes detection of HPV nucleic acids in Caski cells.

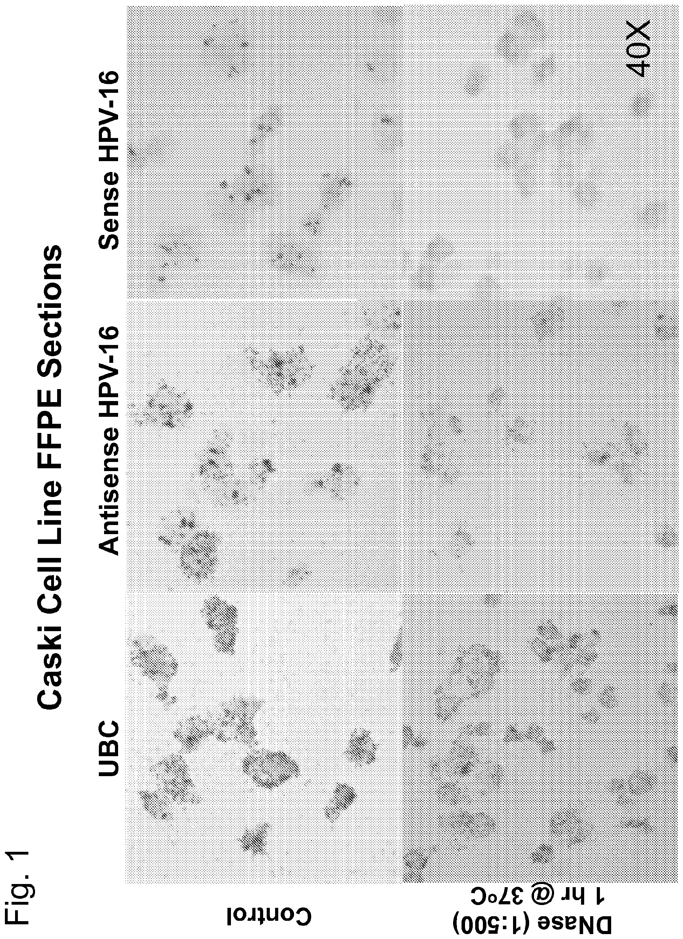

An RNA *in situ* hybridization (ISH) assay was developed based on RNAscope® technology (Advanced Cell Diagnostics) to detect the mRNA of E6 and E7 oncogenes of HR-HPVs (Bishop et al., Am. J. Surg. Pathol. 36:1874-1882 (2012); Schache et al., Br. J. Cancer 108:1332-1339 (2013); Ukpo et al., Am. J. Surg. Pathol. 35:1343-1350 (2011); Wang et al., J. Mol. Diagn. 14:22-29 (2012)). In the assay, frequent nuclear staining nuclear staining was observed, which may be DNA in origin, in HPV-infected cells. To investigate this, RNAscope® ISH assays were performed on the cervical cancer cell line Caski known to harbor HPV16 using probes targeting the E6 and E7 genes. Two types of probes were used: a standard antisense probe complementary to E6 and E7 mRNA, and a sense probe targeting the same regions but in the same sense as the E6/E7 mRNA (probe sets commercially available from Advanced Cell Diagnostics). In other words, the antisense probe can hybridize with E6 and E7 mRNA and the DNA strand having the same sense as the mRNA. Thus, the sense probe was designed to detect only HPV DNA, whereas the antisense probe could detect both mRNA and DNA. Furthermore, the signals from the sense probe should be DNase-sensitive, whereas the signals from the antisense probe should be partially sensitive: the DNA signals should be abolished/diminished by DNase treatment but the mRNA-derived signals should remain. These expected results were observed in Caski cells (Figure 1).

Figure 1 shows serial sections of a formalin-fixed paraffin-embedded block of cultured Caski cells. Caski cells were stained for UBC, encoding ubiquitin (endogenous RNA control) (left column), antisense probe against HPV16 E6/E7 mRNA (middle column), and the sense probe against HPV16 E6/E7 DNA (right column). The top row shows standard conditions (no DNase). In the bottom row, sections were pretreated with DNase then hybridized with HPV probes. RNAscope® staining signals are in brown color (DAB), and nuclei were stained with hematoxylin (blue).

Thus, this experiment demonstrated that the standard antisense probe targeting HPV E6/E7 mRNA assay could actually simultaneously detect both HPV DNA and mRNA. The experiment also demonstrated the ability to use the sense probe as a method for distinguishing DNA- and RNA-derived signals.

These results demonstrate that probes to detect HPV nucleic acids can be used to simultaneously detect both HPV DNA and mRNA in a cell.

### EXAMPLE II

### HPV Detection in Cervical Tissue Samples

This example describes detection of HPV nucleic acids in cervical tissue samples.

High-risk HPV E6/E7 mRNA is known to be up-regulated in high grade CIN lesions (CIN2+) but not in low grade lesions (CIN1)(Lie and Kristensen, Expert Rev. Mol. Diagn. 8:405-415 (2008)). An *in situ hybridization* assay was performed essentially as described in Example I on cervical tissue samples. Briefly, 5 micron thickness slide-mounted sections were deparaffinized through 100% xylene and ethanol washes. Tissues were then treated serially with commercially available buffers (Advanced Cell Diagnostics): Pre-Treatment 1 solution (endogenous hydrogen peroxidase block with Pretreat 1 solution for 10 minutes at room temperature); Pre-Treatment 2 (100°C, 15 minutes immersion in Pretreat 2 solution); and, Pre-Treatment 3 (protease digestion, 40°C for 30 minutes); rinses with water were performed after each Pre-Treatment step. Tissues were then hybridized in HR 18 HPV hybridization solution, without a cover slip, at 40°C for 2 hours in a HybEZ Oven (Advanced Cell Diagnostics, Hayward, CA). After wash buffer steps, signal amplification from the hybridized probe was performed by the serial application of Amp 1 (PreAmplifier step), Amp 2 (signal enhancer step), Amp 3 (amplifier step), Amp 4 (Label Probe step), Amp 5 and Amp 6 (signal amplifications steps); wash buffer steps were performed after each Amp step. HRP activity was then demonstrated by the application of 3,3'-diaminobenzidine (DAB) for 10 minutes at ambient temperature. Sections were then counterstained with hematoxylin, dehydrated through graded ethanol (50%, 70% and 100%) and xylene and then mounted with Cytoseal XYL (Thermo Fisher Scientific Inc., Waltham, MA).

Figure 2 shows HPV E6/E7 staining patterns in normal cervix and HPV-associated cervical lesions of different histological grade. Cervical biopsies were stained for high risk HPV E6/E7 using a pool of probes against 18 subtypes (16, 18, 26, 31, 33, 35, 39, 45, 51, 52, 53, 56, 58, 59, 66, 68, 73 and 82). Normal cervix was completely negative for high-risk HPV. Note that CIN1 had strong staining in the upper 1/3 layer of the cervical epithelium, but its staining pattern is different from that of CIN3, where the signals appeared as fine brown granules throughout the epithelium. Some CIN2 exhibit an intermediate pattern as shown. RNAscope® staining signals are in brown color (DAB), and nuclei were stained with hematoxylin (blue).

Unexpectedly, the RNAscope® HPV E6/E7 mRNA assay detected robust signals in both CIN1 and CIN2+ lesions (Figure 2). However, the signal patterns in CIN1, CIN2 and CIN3 lesions were clearly different. In CIN1, diffuse full nuclear staining was present in many cells but only in the superficial layer of the epithelium, whereas in CIN3, the staining appeared as fine granules primarily in the cytoplasm involving most of the epithelium. In some CIN2 lesions, a hybrid signal pattern was observed; there were strong full nuclear staining in the superficial layer and cytoplasmic signals in the lower epithelium. The unexpected strong staining in CIN1 was expected to be from HPV DNA, similar to what was observed in Caski cells (Figure 1). The diffuse nuclear signals might represent viral DNA produced in the productive phase of the viral life cycle, after HPV genome amplification but before the viral capsids are released from the host cells. Therefore, the CIN1 E6/E7 staining pattern appears to represent a transient HPV infection, whereas the CIN3 E6/E7 pattern appears to represent persistent HPV infection.

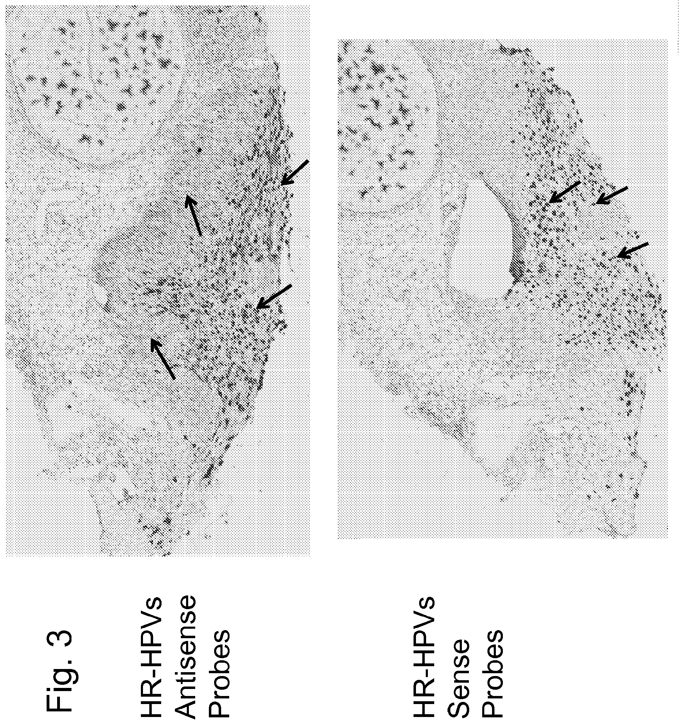

To determine the nature of the signals, sense probes and DNase/RNase treatment were used to confirm that the diffuse nuclear signals in CIN1 and CIN2 lesions were from the antisense probe hybridizing to HPV DNA. In Figure 3, serial sections of a CIN2 lesion were stained with either sense or antisense E6/E7 probes for HR-HPV. Figure 3 shows a CIN2 lesion stained with the antisense probes targeting high-risk HPVs or the corresponding sense probes. RNAscope® staining signals are in brown color (DAB), and nuclei were stained with hematoxylin (blue).

The regular antisense probes detected the "hybrid" signal pattern, with both strong diffuse nuclear staining in the superficial layer (Figure 3, leftward pointing arrows) and fine granular staining in both the cytoplasm and the nucleus (Figure 3, rightward pointing arrows). However, the sense probes detected only the strong nuclear staining in the upper part of the epithelium, which appeared strikingly similar to that seen with the antisense probes in the same region of the lesion. These data provided preliminary support that the diffuse nuclear staining in CIN1 and CIN2 lesions seen with the antisense probe might be from HPV DNA by virtue of its detection by the sense probes as well.

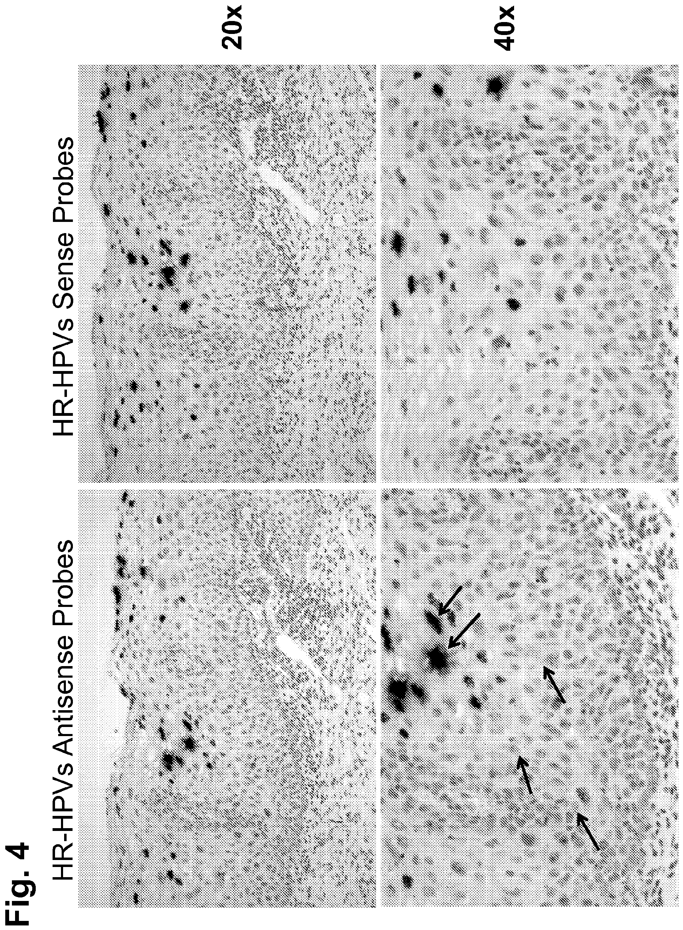

In an independent experiment, DNase and RNase were used to further confirm the DNA or RNA nature of the observed signals in a CIN2 lesion. Figure 4 shows the staining results with antisense and sense probes. Figure 4 shows a CIN2 lesion stained with the antisense probes targeting high-risk HPVs or the corresponding sense probes. The upper panels are 20x lens; the lower panels are 40X lens. RNAscope® signals are in brown color (DAB), and nuclei were stained with hematoxylin (blue).

Strong diffuse nuclear staining was observed in the upper superficial layer of the epithelium from both probes (Figure 4, leftward pointing arrows). The anti-sense probe also detected additional cytoplasmic staining in the basal layer, which could be seen more clearly at higher magnification (40X)(Figure 4, rightward pointing errors).

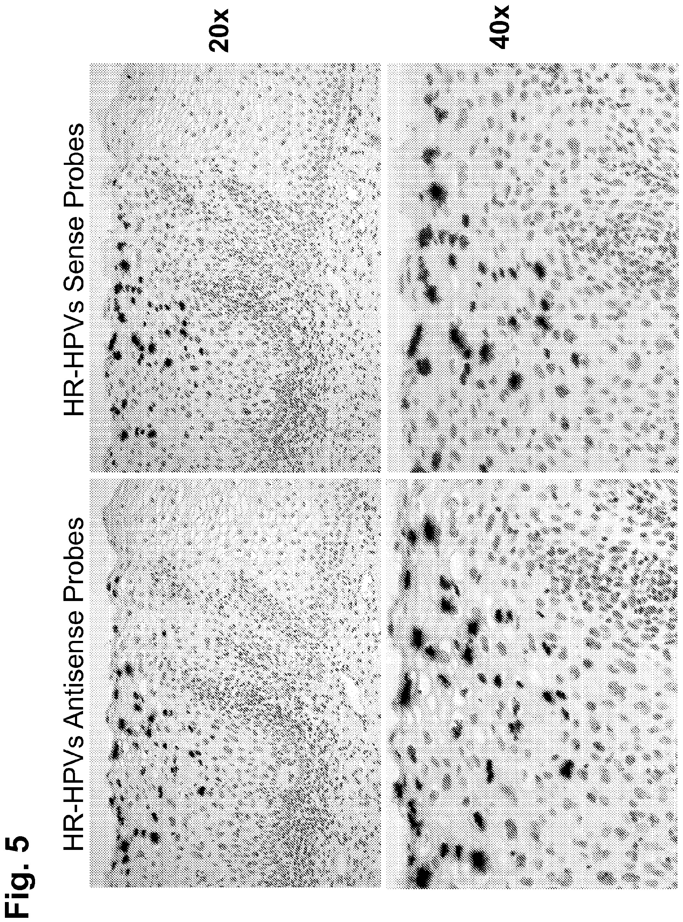

Figure 5 shows the same CIN2 lesion as in Figure 3 pretreated with RNase then stained with the antisense probes targeting high-risk HPVs or the corresponding sense probes. The upper panels are 20x lens; the lower panels are 40X lens. RNAscope® signals are in brown color (DAB), and nuclei are stained with hematoxylin (blue). Pre-treatment with RNase had no impact on the strong full nuclear staining in the upper layer with either antisense or sense probes but did abolish the weaker fine grained cytoplasmic signals in the basal layer observed using the anti-sense probes, thus confirming the RNA nature of the latter signals (Figure 5).

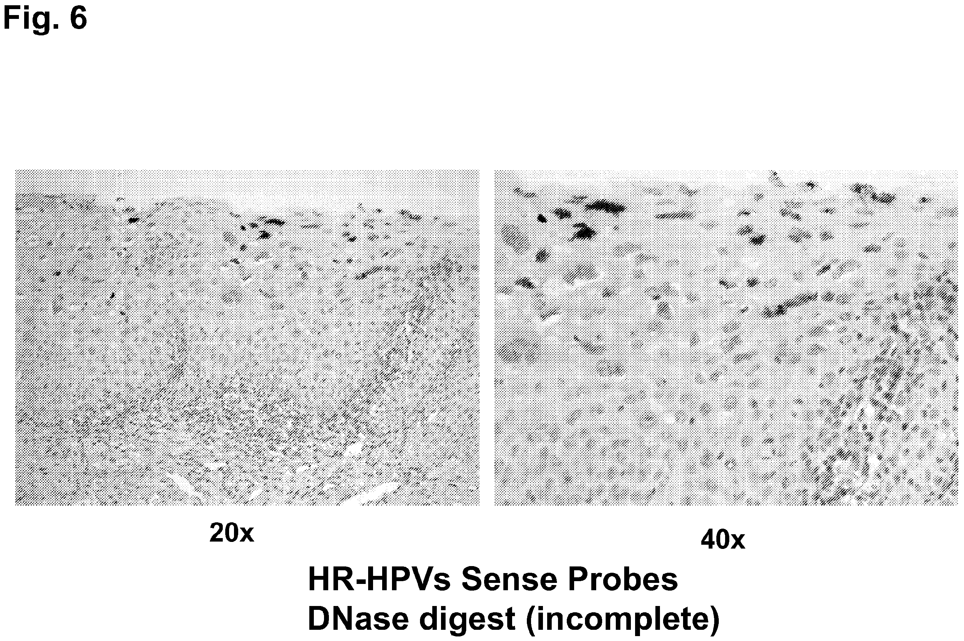

Figure 6 shows the same CIN2 lesion as in Figure 3 pretreated with DNase then stained with the sense HPV probes. The left panel is 20x lens; the right panel 40X lens. RNAscope® signals are in brown color (DAB), and nuclei were stained with hematoxylin (blue). Pre-treatment with DNase caused a significant reduction in the number of diffusely stained cells in the upper layer observed with the sense probe (Figure 6). The remaining signals were likely due to incomplete DNase digestion. The DNase treatment experiment together with the use of sense probes thus confirmed that the strong full nuclear signals in the upper layer of this lesion were from HPV DNA.

The signal patterns detected by the sense probes in RNAscope® ISH assays were similar to those reported previously using conventional DNA *in situ* hybridization techniques (De Marchi Triglia et al., Gynecol. Oncol. 112(1), 114-118(2009); Evans et al., Mod. Pathol. 15:1339-1347 (2002)). However, simultaneous detection of both HPV DNA and RNA by the same probe has not been reported.

These results demonstrate that detection of HPV nucleic acids, both DNA and mRNA, can be detected simultaneously in the same assay and can be used to stratify and categorize cervical tissue samples.

## Claims

1. A method of categorizing a cervical tissue sample, comprising:
(a) performing an *in situ* hybridization assay using an antisense E6 or E7 probe on a cervical tissue sample, wherein the antisense E6 or E7 probe can simultaneously detect high-risk HPV DNA and HPV RNA;
(b) detecting the presence of HPV nucleic acid, wherein said detecting comprises detecting HPV DNA and HPV RNA; and
(c) categorizing the cervical tissue sample,
wherein
(i) the presence of diffuse nuclear staining of HPV nucleic acid only in the superficial layer of the epithelium of the cervical sample indicates a transient HPV infection state,
(ii) the presence of granular staining of HPV nucleic acid in the cytoplasm and/or the nucleus in the intermediate and basal layers of the epithelium of the cervical sample indicates a persistent HPV infection state, or
(iii) the presence of diffuse nuclear staining of HPV nucleic acid in the superficial layer of the epithelium and granular staining of HPV nucleic acid in the cytoplasm and/or the nucleus of the intermediate layer of the epithelium of the cervical sample indicates a transition HPV infection state.

2. The method of claim 1, wherein the transition HPV infection state indicates that the HPV virus starts to establish persistence in the basal layer.

3. The method of claim 1, wherein:
(i) detection of HPV DNA only in the superficial layer of the epithelium of the cervical tissue sample indicates a low-grade lesion;
(ii) detection of HPV RNA in the intermediate and basal layers of the epithelium of the cervical tissue sample indicates a high-grade lesion;
(iii) detection of HPV RNA in the basal or intermediate layers and HPV DNA in the superficial layer of the epithelium of the cervical tissue sample indicates high grade lesion; or
(iv) detection of HPV RNA indicates a high grade lesion.

4. A method of determining the prognosis of patient outcome based on analysis of a cervical tissue sample, comprising performing the method of claim 1.

5. The method of claim 4, wherein:
(i) determination of a transient HPV infection state indicates an increased likelihood of clearing the HPV infection;
(ii) determination of a persistent infection state indicates an increased likelihood of progressing to a cancerous state; or
(iii) determination of a transition HPV infection state indicates an increased likelihood of progressing to a cancerous state.

6. The method of any one of claims 1-5, wherein the antisense E6 or E7 probe can detect high-risk HPV selected from one or more of the HPV subtypes 16, 18, 26, 31, 33, 35, 39, 45, 51, 52, 53, 56, 58, 59, 66, 68, 73 and 82.

7. The method of any one of claims 1-6, wherein the sample is pretreated to denature double stranded DNA prior to performing the *in situ* hybridization assay.

8. A method of categorizing a cervical cytology sample, comprising:
(a) performing an *in situ* hybridization assay using an antisense E6 or E7 probe on a cervical cytology sample, wherein the antisense E6 or E7 probe can simultaneously detect high-risk HPV DNA and HPV RNA;
(b) detecting the presence of HPV nucleic acid, wherein said detecting comprises detecting HPV DNA and HPV RNA; and
(c) categorizing the cervical cytology sample,
wherein:
(i) the presence of only diffuse nuclear staining of HPV nucleic acid in HPV positive cells of the cervical cytology sample indicates that the sample corresponds to a transient HPV infection state;
(ii) the presence of granular staining of HPV nucleic acid in the cytoplasm and/or nucleus in HPV positive cells of the cervical cytology sample indicates that the sample corresponds to a persistent HPV infection state; or
(iii) the presence of a mixture of HPV positive cells having granular staining of HPV nucleic acid in the cytoplasm and/or nucleus and cells having diffuse nuclear staining of HPV nucleic acid indicates that the sample corresponds to a transition HPV infection state.

9. The method of claim 8, wherein the transition HPV infection state indicates that the HPV virus starts to establish persistence in the basal layer.

10. A method of determining the prognosis of patient outcome based on analysis of a cervical cytology sample, comprising performing the method of claim 8.

11. The method of claim 10, wherein:
(i) determination of a transient HPV infection state indicates an increased likelihood of clearing the HPV infection;
(ii) determination of a persistent infection state indicates an increased likelihood of progressing to a cancerous state; and
(iii) determination of a transition HPV infection state indicates an increased likelihood of progressing to a cancerous state.

12. The method of any one of claims 8-11, wherein the antisense E6 or E7 probe can detect high risk HPV selected from one or more of the HPV subtypes 16, 18, 26, 31, 33, 35, 39, 45, 51, 52, 53, 56, 58, 59, 66, 68, 73 and 82.

13. The method of any one of claims 8-12, wherein the sample is pretreated to denature double stranded DNA prior to performing the *in situ* hybridization assay.

## Patentansprüche

1. Verfahren zur Kategorisierung einer zervikalen Gewebeprobe, umfassend:
(a) Durchführen eines *in-situ-*Hybridisierungsassays unter Verwendung einer Antisense-E6- oder E7-Sonde an einer zervikalen Gewebeprobe, wobei die Antisense-E6- oder E7-Sonde gleichzeitig Hochrisiko-HPV-DNA und HPV-RNA nachweisen kann;
(b) Detektieren der Anwesenheit von HPV-Nukleinsäure, wobei das Detektieren das Detektieren von HPV-DNA und HPV-RNA umfasst; und
(c) Kategorisieren der zervikalen Gewebeprobe, wobei
(i) das Vorhandensein einer diffusen nukleären Färbung von HPV-Nukleinsäure nur in der oberflächlichen Schicht des Epithels der zervikalen Probe einen transienten HPV-Infektionszustand anzeigt,
(ii) das Vorhandensein einer granulären Färbung von HPV-Nukleinsäure im Zytoplasma und/oder im Nukleus in den mittleren und basalen Schichten des Epithels der zervikalen Probe einen persistenten HPV-Infektionszustand anzeigt, oder
(iii) das Vorhandensein einer diffusen nukleären Färbung von HPV-Nukleinsäure in der oberflächlichen Schicht des Epithels und einer granulären Färbung von HPV-Nukleinsäure im Zytoplasma und/oder dem Kern der mittleren Schicht des Epithels der zervikalen Probe einen transienten HPV-Infektionszustand indiziert.

2. Verfahren nach Anspruch 1, wobei der transiente HPV-Infektionszustand indiziert, dass das HPV-Virus beginnt, Persistenz in der Basalschicht zu etablieren.

3. Verfahren nach Anspruch 1, wobei:
(i) die Detektion von HPV-DNA nur in der oberflächlichen Schicht des Epithels der zervikalen Gewebeprobe eine niedriggradige Läsion indiziert;
(ii) die Detektion von HPV-RNA in den mittleren und basalen Schichten des Epithels der zervikalen Gewebeprobe eine hochgradige Läsion indiziert;
(iii) die Detektion von HPV-RNA in den basalen oder intermediären Schichten und HPV-DNA in der oberflächlichen Schicht des Epithels der zervikalen Gewebeprobe eine hochgradige Läsion indiziert; oder
(iv) die Detektion von HPV-RNA eine hochgradige Läsion indiziert.

4. Verfahren zur Bestimmung der Prognose des Behandlungsergebnisses basierend auf der Analyse einer zervikalen Gewebeprobe, umfassend die Durchführung des Verfahrens nach Anspruch 1.

5. Verfahren nach Anspruch 4, wobei:
(i) die Bestimmung eines transienten HPV-Infektionszustands eine erhöhte Wahrscheinlichkeit indiziert, die HPV-Infektion zu überwinden;
(ii) die Bestimmung eines persistierenden Infektionszustands eine erhöhte Wahrscheinlichkeit indiziert, zu einem kanzerösen Zustand fortzuschreiten; oder
(iii) die Bestimmung eines transienten HPV-Infektionszustands eine erhöhte Wahrscheinlichkeit indiziert, zu einem kanzerösen Zustand fortzuschreiten.

6. Verfahren nach einem der Ansprüche 1-5, wobei die Antisense-E6- oder E7-Sonde Hochrisiko-HPV detektieren kann, das aus einem oder mehreren der IPV-Subtypen 16, 18, 26, 31, 33, 35, 39, 45, 51, 52, 53, 56, 58, 59, 66, 68, 73 und 82 ausgewählt ist.

7. Verfahren nach einem der Ansprüche 1 bis 6, wobei die Probe vorbehandelt wird, um doppelsträngige DNA vor der Durchführung des *in-situ-*Hybridisierungsassays zu denaturieren.

8. Verfahren zur Kategorisierung einer zervikalen Zytologieprobe, umfassend:
(a) Durchführen eines *in situ*-Hybridisierungsassays unter Verwendung einer Antisense-E6- oder E7-Sonde an einer zervikalen Zytologieprobe, wobei die Antisense-E6- oder E7-Sonde gleichzeitig Hochrisiko-HPV-DNA und HPV-RNA detektieren kann;
(b) Detektieren der Anwesenheit von HPV-Nukleinsäure, wobei das Detektieren das Detektieren von HPV-DNA und HPV-RNA umfasst; und
(c) Kategorisieren der zervikalen Zytologieprobe, wobei:
(i) das Vorhandensein von nur diffuser nukleärer Färbung von HPV-Nukleinsäure in HPV-positiven Zellen der zervikalen Zytologieprobe indiziert, dass die Probe einem transienten HPV-Infektionszustand entspricht,
(ii) das Vorhandensein von granulärer Färbung von HPV-Nukleinsäure im Zytoplasma und/oder Nukleus in HPV-positiven Zellen der zervikalen Zytologieprobe indiziert, dass die Probe einem persistenten HPV-Infektionszustand entspricht; oder
(iii) das Vorhandensein einer Mischung aus HPV-positiven Zellen mit granulärer Färbung von HPV-Nukleinsäure im Zytoplasma und/oder Kern und Zellen mit diffuser Kernfärbung von HPV-Nukleinsäure indiziert, dass die Probe einem transienten HPV-Infektionszustand entspricht.

9. Verfahren nach Anspruch 8, wobei der transiente HPV-Infektionszustand indiziert, dass das der HPV-Virus beginnt, Persistenz in der Basalschicht zu etablieren.

10. Verfahren zur Bestimmung der Prognose des Krankheitsverlaufs bei einem Patienten basierend auf der Analyse einer zervikalen Zytologieprobe, umfassend die Durchführung des Verfahrens nach Anspruch 8.

11. Verfahren nach Anspruch 10, wobei:
(i) die Bestimmung eines transienten HPV-Infektionszustands eine erhöhte Wahrscheinlichkeit indiziert, die HPV-Infektion zu überwinden;
(ii) die Bestimmung eines persistenten Infektionszustands eine erhöhte Wahrscheinlichkeit indiziert, zu einem kanzerösen Zustand fortzuschreiten; und
(iii) die Bestimmung eines Übergangs-HPV-Infektionszustands eine erhöhte Wahrscheinlichkeit indiziert, zu einem kanzerösen Zustand fortzuschreiten.

12. Verfahren nach einem der Ansprüche 8-11, wobei die Antisense-E6- oder E7-Sonde Hochrisiko-HPV, ausgewählt aus einem oder mehreren der HPV-Subtypen 16, 18, 26, 31, 33, 35, 39, 45, 51, 52, 53, 56, 58, 59, 66, 68, 73 und 82, nachweisen kann.

13. Verfahren nach einem der Ansprüche 8 bis 12, wobei die Probe vorbehandelt wird, um doppelsträngige DNA vor der Durchführung des *in-*situ-Hybridisierungsassays zu denaturieren.

## Revendications

1. Procédé pour catégoriser un échantillon de tissu cervical, comprenant :
(a) la réalisation d'un essai d'hybridation *in situ* utilisant une sonde E6 ou E7 antisens sur un échantillon de tissu cervical, dans lequel la sonde E6 ou E7 antisens peut détecter simultanément l'ADN du VPH et l'ARN du VPH à haut risque ;
(b) la détection de la présence de l'acide nucléique du VPH, dans lequel ladite détection comprend la détection de l'ADN du VPH et de l'ARN du VPH ; et
(c) la catégorisation de l'échantillon de tissu cérébral,
dans lequel
(i) la présence d'une coloration nucléaire diffuse de l'acide nucléique du VPH uniquement dans la couche superficielle de l'épithélium de l'échantillon cervical indique un état transitoire d'infection par le VPH,
(ii) la présence d'une coloration granulaire de l'acide nucléique du VPH dans le cytoplasme et/ou le noyau dans les couches intermédiaires et basales de l'épithélium de l'échantillon cervical indique un état persistant d'infection par le VPH, ou
(iii) la présence d'une coloration nucléaire diffuse de l'acide nucléique du VPH dans la couche superficielle de l'épithélium et d'une coloration granulaire de l'acide nucléique du VPH dans le cytoplasme et/ou le noyau de la couche intermédiaire de l'épithélium de l'échantillon cervical indique un état de transition d'infection par le VPH.

2. Procédé selon la revendication 1, dans lequel l'état de transition d'infection du VPH indique que le virus VPH commence à établir une persistance dans la couche basale.

3. Procédé selon la revendication 1, dans lequel
(i) la détection de l'ADN du VPH uniquement dans la couche superficielle de l'épithélium de l'échantillon de tissu cervical indique une lésion de grade faible,
(ii) la détection de l'ARN du VPH dans les couches intermédiaires et basales de l'épithélium de l'échantillon de tissu cervical indique une lésion de grade élevé ;
(iii) la détection de l'ARN du VPH dans les couches basale et intermédiaire et de l'ADN du VPH dans la couche superficielle de l'épithélium de l'échantillon de tissu cervical indique une lésion de grade élevé ; ou
(iv) la détection de l'ARN du VPH indique une lésion de grade élevé.

4. Procédé de détermination du pronostic de l'évaluation de l'état de santé d'un patient sur la base de l'analyse d'un échantillon de tissu cervical, comprenant la mise en œuvre du procédé selon la revendication 1.

5. Procédé selon la revendication 4, dans lequel :
(i) la détermination d'un état transitoire d'infection par le VPH indique une probabilité accrue d'élimination de l'infection par le VPH ;
(ii) la détermination d'un état persistant d'infection indique une probabilité accrue d'évolution vers un état cancéreux ; ou
(iii) la détermination d'un état de transition d'infection par le VPH indique une probabilité accrue d'évolution vers un état cancéreux.

6. Procédé selon l'une quelconque des revendications 1 à 5, dans lequel la sonde E6 ou E7 antisens peut détecter un VPH à haut risque choisi parmi un ou plusieurs des sous-types de VPH 16, 18, 26, 31, 33, 35, 39, 45, 51, 52, 53, 56, 58, 59, 66, 68, 73 et 82.

7. Procédé selon l'une quelconque des revendications 1 à 6, dans lequel l'échantillon est prétraité pour dénaturer l'ADN double brin avant la mise en œuvre de l'essai d'hybridation *in situ.*

8. Procédé pour catégoriser un échantillon de cytologie cervicale, comprenant :
(a) la mise en œuvre d'un essai d'hybridation *in situ* utilisant une sonde E6 ou E7 antisens sur un échantillon de cytologie cervicale, dans lequel la sonde E6 ou E7 antisens peut détecter simultanément l'ADN du VPH et l'ARN du VPH à haut risque ;
(b) la détection de la présence de l'acide nucléique du VPH, dans lequel ladite détection comprend la détection de l'ADN du VPH et de l'ARN du VPH ; et
(c) la catégorisation de l'échantillon de cytologie cervicale,
dans lequel
(i) la présence seulement d'une coloration nucléaire diffuse de l'acide nucléique du VPH dans les cellules VPH positives de l'échantillon de cytologie cervicale indique que l'échantillon correspond à un état transitoire d'infection par le VPH ;
(ii) la présence d'une coloration granulaire de l'acide nucléique du VPH dans le cytoplasme et/ou le noyau de cellules VPH positives de l'échantillon de cytologie cervicale indique que l'échantillon correspond à un état persistant d'infection par le VPH, ou
(iii) la présence d'un mélange de cellules VPH positives ayant une coloration granulaire de l'acide nucléique du VPH dans le cytoplasme et/ou le noyau et de cellules ayant une coloration nucléaire diffuse de l'acide nucléique du VPH indique que l'échantillon correspond à un état de transition de l'infection par le VPH.

9. Procédé selon la revendication 8, dans lequel l'état de transition de l'infection par le VPH indique que le virus VPH commence à établir une persistance dans la couche basale.

10. Procédé de détermination du pronostic de l'évolution de l'état de santé d'un patient sur la base de l'analyse d'un échantillon de cytologie cervicale, comprenant la mise en œuvre du procédé selon la revendication 8.

11. Procédé selon la revendication 10, dans lequel :
(i) la détermination d'un état transitoire d'infection par le VPH indique une probabilité accrue d'élimination de l'infection par le VPH ;
(ii) la détermination d'un état persistant d'infection indique une probabilité accrue d'évolution vers un état cancéreux ; et
(iii) la détermination d'un état de transition de l'infection par le VPH indique une probabilité accrue d'évolution vers un état cancéreux.

12. Procédé selon l'une quelconque des revendications 8 à 11, dans lequel la sonde E6 ou E7 peut détecter un VPH à haut risque choisi parmi un ou plusieurs des sous-types du VPH 16, 18, 26, 31, 33, 35, 39, 45, 51, 52, 53, 56, 58, 59, 66, 68, 73 et 82.

13. Procédé selon l'une quelconque des revendications 8 à 12, dans lequel l'échantillon est prétraité pour dénaturer l'ADN double brin avant mise en œuvre de l'essai d'hybridation *in situ.*
